# EUROPÄISCHE PATENTSCHRIFT

(11) **EP 1 141 187 B1**
(45) Veröffentlichungstag und Bekanntmachung des Hinweises auf die Patenterteilung: **02.03.2005**
(21) Anmeldenummer: 00965841.0
(22) Anmeldetag: 13.09.2000
(51) Int. Cl.: C11D 1/28, C11D 1/10, C11D 1/34, C11D 1/04, C11D 1/90, C11D 1/52, A61K 7/50

(54) **TENSIDZUSAMMENSETZUNG ENTHALTEND GEMINITENSIDE UND CO-AMPHIPHILE, IHRE HERSTELLUNG UND IHRE VERWENDUNG**
SURFACTANT COMPOSITION CONTAINING GEMINI SURFACTANTS AND CO-AMPHIPHILES, PRODUCTION AND USE THEREOF
COMPOSITION TENSIOACTIVE CONTENANT DES TENSIOACTIFS GEMINI ET DES CO-AMPHIPHILES, SA PRODUCTION ET SON UTILISATION

(30) Priorität: 13.09.1999 DE 19943668
(43) Veröffentlichungstag der Anmeldung: 10.10.2001
(73) Patentinhaber: SASOL Germany GmbH, 22297 Hamburg (DE)
(72) Erfinder: DAHMS, Gerd, H., D-47138 Duisburg (DE); KWETKAT, Klaus, D-59192 Bergkamen (DE)
(74) Vertreter: Schupfner, Georg U.
(86) Internationale Anmeldenummer: PCT/DE2000/003162
(87) Internationale Veröffentlichungsnummer: WO 2001/019945

(56) Entgegenhaltungen:
- EP-A- 0 697 244
- EP-A- 0 697 245
- WO-A-97/46513
- WO-A-98/20853
- DE-A- 19 505 368
- DE-A- 19 608 117
- DE-A- 19 750 246
- DE-A- 19 855 080

## Beschreibung

Die Erfindung betrifft eine Tensidzusammensetzung aus Geminitensiden und Co-Amphiphil(en), ein Verfahren zu deren Herstellung und ihre Verwendung, insbesondere als Emulgier- und Dispergiermittel.

Mischungen aus Tensiden und Co-Amphiphilen und deren Verwendung in Emulsionen oder Dispersionen sind bekannt. Aufgabe dieser Mischungen ist die Anordnung der Tensidmoleküle an den Phasengrenzschichten zu optimieren. Bei der Herstellung von Emulsionen ist oft beabsichtigt, flüssigkristalline lamellare Phasen auszubilden, die zur Stabilisierung von Emulsionen genutzt werden können.

Je nach Anwendungen werden aufeinander abgestimmte Mischungen spezieller Tenside und Co-Amphiphile eingesetzt. Für Anwendungen, bei denen hohe Elektrolytgehalte in wäßrigen Lösungen bzw. Emulsionen oder Dispersionen eingesetzt werden müssen, gelten ionische Tenside bisher als weitgehend ungeeignet. Um eine leichtere Auswahl geeigneter Tenside zu ermöglichen, versucht man, deren Polarität zu quantifizieren bzw. zu klassifizieren und damit letztlich Aussagen über deren Eigenschaften und Anwendungsprofile zu machen. Das HLB-Konzept in seinen verschiedenen Ausprägungen sei hier exemplarisch als einfache Variante dieses Bestrebens erwähnt.

Geminitenside sind in ihren nichtionischen und kationischen Varianten seit mehr als zwanzig Jahren bekannt. Größeres Interesse haben sie allerdings erst Anfang der neunziger Jahre gefunden. Die Veröffentlichung von R. Zana "Dimeric (Gemini) Surfactants" in Novel Surfactants Preparation, Applications and Biodegradability, C. Holmberg (ed.), Marcel Dekker, (1998) 241, gibt eine Übersicht über den Stand der Technik.

Gegenstand der meisten Veröffentlichungen der jüngeren Vergangenheit sind ionische Geminitenside, da diese eine sprunghafte Verbesserung der Grenzflächenaktivität, gemessen z.B. anhand der kritischen Mizellbildungskonstante oder der Senkung der Oberflächenspannung von Wasser, im Vergleich zu konventionellen Tensiden möglich machten.

Geminitenside sind zunächst im wesentlichen zur Verwendung in Wasch- und Reinigungsmitteln empfohlen worden.

In JP-A 08/268 865 wird der Einsatz von Geminitensiden auch in kosmetischen Formulierungen beschrieben. Dort sind Formulierungen vorgeschlagen worden, in denen konventionelle, anionische Tenside durch ebenfalls anionische, aber deutlich hautmildere Geminitenside ohne besondere Anpassung der Formulierungen ausgetauscht wurden.

In der EP-A-0 697 244 werden amphotere Geminitenside beschrieben, die auch als Mischung mit anderen anionischen, nichtionischen, kationischen oder amphoteren Tensiden eingesetzt werden können; als Verwendung ist der Einsatz in Reinigungsmitteln offenbart. Aus der WO 95/19953 sind Geminitenside (Geminiamide) bekannt, die als Komponente u.a. in üblichen Reinigungsmittel-Zusammensetzungen verwendet werden können. Die WO 95/19955 nennt Geminipolyether als eine weitere Klasse von Geminitensiden, die ebenfalls auf dem vorstehenden Anwendungsgebiet eingesetzt werden können. Gemische von Geminitensiden des alkoxylierten Bisalkylphenol-Typs mit anderen Tensiden sind aus der WO 97/23449 bekannt.

In der JP-A 11/60430 und JP-A 11/60437 wird der Einsatz von anionischen Geminitensiden in Kosmetika beschrieben, wobei diese auch gemeinsam mit anderen Tensiden vorliegen können.

Die direkte Substitution konventioneller Tenside durch Geminitenside führt jedoch nicht, wie aufgrund der großen Verbesserung der Grenzflächenaktivität möglicherweise hätte erwartet werden können, zu signifikanten Effizienzsteigerungen oder anderen, die Eigenschaften der Anwendungsformulierungen verbessernden Effekten. Der geringe Vorteil der Tensid-Substitution rechtfertigt den Aufwand, der durch den Einbau eines weiteren Strukturelements in die Grundstruktur entsteht, nicht.

Aufgabe der vorliegenden Erfindung ist es, eine Zusammensetzung enthaltend Geminitenside und Hilfsstoffe, im folgenden als Tensidzusammensetzung bezeichnet, bereitzustellen, die es ermöglicht, die aus bestimmten Anwendungen bekannten Vorteile von Geminitensiden für ein möglichst breites Anwendungsspektrum nutzbar zu machen und damit für ihre Anwendung einen breiteren großtechnisch interessanten Bereich zu erschließen.

Diese Aufgabe wird erfindungsgemäß gelöst durch Tensidzusammensetzungen **gemäß Anspruch 1.** Bevorzugte Ausführungsformen der Erfindung sind in den Unteransprüchen dargestellt.

Es wurde festgestellt, daß die erfindungsgemäßen Tensidzusammensetzungen enthaltend Geminitenside und Co-Amphiphile nicht nur punktuell zu einer Optimierung der Anwendungseigenschaften führen, sondern eine hohe Multifunktionalität aufweisen und darüber hinaus auch noch eine höhere Wirksamkeit im Vergleich zu Mischungen aus konventionellen (Nicht-Gemini-)Tensiden und Co-Amphiphilen zeigen.

Die Multifunktionalität äußert sich z.B. darin, daß es überraschend möglich wird, mit ein und derselben Mischung aus Gemini- und Co-Tensid ein hydrophiles Pigment in einer Ölphase wie auch in einer Wasserphase oder auch ein hydrophobes Pigment in einer Ölphase oder in einer Wasserphase zu dispergieren (Wirkung als Emulgator und Dispergator). Bei einigen erfindungsgemäßen Tensidzusammensetzung geht die Multifunktionalität sogar so weit, daß man mit ein und derselben Tensidzusammensetzung sowohl hervorragend emulgieren wie auch dispergieren kann, ohne das verwendete Emulgator- respektive Dispergatorsystem an das jeweilige Anwendungsproblem anpassen zu müssen. Der erfingungsgemäße Tensidzusammensetzung kann hinsichtlich Ihrer Wirkung als Emulgator als Gelnetzwerkemulgator bezeichnet werden.

Die mit Hilfe der erfindungsgemäßen Tensidzusammensetzungen hergestellten O/W-Emulsionen zeichnen sich bei Einhaltung der "Phase Transfer Temperature" - Methode bei ihrer Herstellung durch eine um Größenordnungen geringere Öltröpfchengröße aus.

Die erfindungsgemäßen Tensidzusammensetzungen verleihen mit ihnen hergestellten Emulsionen oder Dispersionen eine bisher auch bei nichtionischen Systemen unbekannte Elektrolytstabilität. Vergleicht man mit bekannten Komplexemulgatoren, so führt eine um den Faktor 5 bis 10 erhöhte Menge an Elektrolyt, die zum Zusammenbruch konventioneller Emulsionen führt, bei Emulsionen, die erfindungsgemäße Komponenten enthalten, nicht zum Zusammenbruch der Emulsion. Die aus den erfindungsgemäßen Tensidzusammensetzungen hergestellten Emulsionen oder Dispersionen bleiben in einem pH-Wert-Bereich von 3 bis 12 ohne weiteres stabil.

Die erfindungsgemäßen Tensidzusammensetzungen weisen darüber hinaus filmbildende Eigenschaften auf, was sich als sehr günstig für ihre vielseitige Verwendung erweist, besonders bei Anwendungen in Cremes zur Hautpflege, beim UV-Schutz oder bei Haarpflege-Anwendungen.

Zu der rein technischen Funktionalität kommt eine außerordentliche Milde. So läßt sich durch Zusatz der erfindungsgemäßen Tensidzusammensetzungen zu Alkylethersulfaten, Alkylbenzolsulfonaten und anderen anionischen Tensiden deren Irritationspotential signifikant herabsetzen. Die Tensidzusammensetzungen zeichnen sich ebenfalls dadurch aus, daß sie eine erhebliche Affinität zu den verschiedensten Grenzflächen zeigen. Je nach Anwendung führt das neben den formulierungstechnischen Vorteilen zu weiteren Anwendungsvorteilen wie der Verbesserung der Naß- und Trockenkämmbarkeit bei Einsatz in Haaren, einer antistatischen Ausrüstung und zu einem seidigen Hautgefühl.

Die überraschend aufgefundene Multifunktionalität bzw. universelle Einsetzbarkeit ist sowohl für einzelne Tenside als auch für Kombinationen konventioneller Tenside und Co-Tenside bisher unbekannt und erlaubt eine deutliche Vereinfachung von Formulierungen, eine Verkürzung der Formulierungsentwicklung und eine Verbesserung der zugehörigen Logistik. Darüber hinaus werden wesentlich leistungsfähigere Anwendungsformulierungen zugänglich.

Unter Geminitensiden versteht man - im Rahmen der vorliegenden Erfindung - grenzflächenaktive Verbindungen, die aus mindestens zwei, Tensideinheiten, d.h. aus hydrophiler Kopfgruppe und hydrophober Gruppe, bestehen, die durch zumindest einen, vorzugsweise einen, Abstandhalter, Spacer genannt, in der Nähe der Kopfgruppe miteinander verknüpft sind. Die Gemini- oder Zwillingstenside werden auch Dimertenside genannt und tragen ihren Namen aufgrund ihrer besonderen Bauart. Abhängig von der Natur der Kopfgruppe gibt es anionische, nichtionische, kationische und amphotere Geminitenside. Anders als konventionelle Tenside, die man ebenfalls in diese Gruppen einteilt, können Geminitenside jedoch auch Kombinationen von Kopfgruppen unterschiedlichen Charakters tragen. Dabei handelt es sich meist um Kombinationen aus nichtionischen und ionischen Gruppen.

Gegenstand der Erfindung sind Tensidzusammensetzungen enthaltend anionische, kationische und/oder neutrale Geminitenside. Bei Kombinationen von ionischen mit nichtionischen Kopfgruppen überwiegt die Natur der ionischen Kopfgruppe im resultierenden Geminitensid, so daß Kombinationen aus nichtionischer und anionischer Kopfgruppe als anionisches Geminitensid klassifiziert werden. Analoges gilt für die Kombinationen nichtionischer mit kationischen oder amphoteren Kopfgruppen. Für die erfindungsgemäßen Tensidzusammensetzungen ist in erster Linie die Morphologie, also die relative Anordnung der verschiedenen Struktureinheiten (hydrophile Gruppen, Spacer, hydrophobe Ketten) zueinander, entscheidend und nicht die Art der Kopfgruppe. Die erfindungsgemäß eingesetzten Geminitenside weisen demnach folgende Struktur auf:

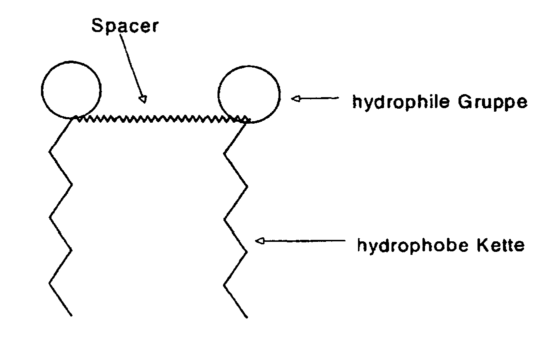

Zu den bevorzugt in den erfindungsgemäßen Tensidzusammensetzungen eingesetzten Geminitensiden zählen solche, die an der Verknüpfungsstelle zwischen Spacer, hydrophiler und hydrophober Gruppe Stickstoffatome enthalten. Dazu gehören bevorzugt Geminitenside mit Amin- oder Amidgruppen aufweisenden Spacern aber auch sich von Dicarbonsäuren ableitende Spacer, sich von Betainstrukturen ableitende, hydrophile Doppelkopfgruppen, die gegebenenfalls durch Alkoxylieren (insbesondere Ethoxylieren) hergestellte Seitengruppen aufweisen, die Sulfonsäure-, Phosphonsäure-, Carbonsäure oder Alkoholgruppen (einschließlich Polyalkoholen) tragen können, und jeweils hydrophobe Ketten mit 5 bis 25 C-Atomen aufweisen, die verzweigt oder unverzweigt sein können und bis zu 2 nicht benachbarte Doppelbindungen tragen können.

Die folgenden Strukturvarianten der Geminitenside sind für die erfindungsgemäßen Tensidzusammensetzungen besonders geeignet.

### Variante A: Auf amid- oder aminhaltigen Spacern beruhende Strukturen

### A.I Geminitenside der allgemeinen Formel (A.I) analog WO 96/14926

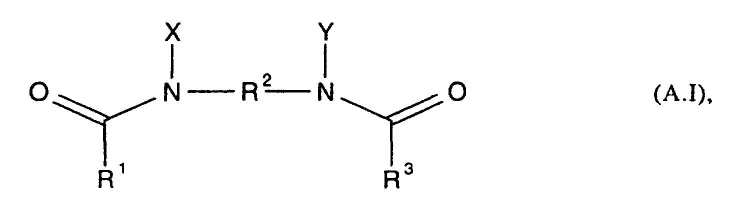

wobei die Substituenten folgende Bedeutung haben:
- R¹, R³: C₅- bis C₂₅-Alkyl, verzweigt oder unverzweigt, gesättigt, gegebenenfalls bis zu 2-fach nicht-benachbart ungesättigt;
- R²: C₁- bis C₁₂-Alkylen;
- X,Y: (C₂H₄O-)ₓ(C₃H₆O-)_{y}-FR; x+y ≥ 1, x: 0-15, y: 0-10 und
- FR: -SO₃M, -CH₂-CO₂M, -P(O)(OM)₂, H, -C₃H₆SO₃M; oder -CH₂(CHOH)₄CH₂OH, soweit x+y=0; wobei M = Alkali, (Alkyl)Ammonium, Alkanolammonium, H oder ½ Erdalkali ist.

### A.II Geminitenside mit Dicarbonsäure-stämmigen Spacern der allgemeinen Formel (A.II) analog WO 96/25388

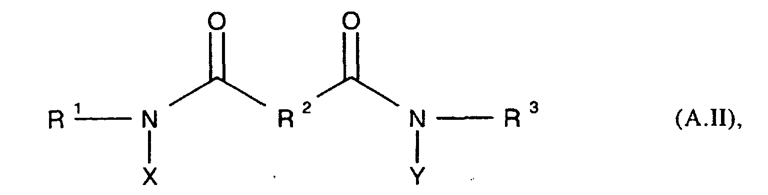

wobei die Substituenten die für die allgemeine Formel (A.I) angegebene Bedeutung haben.

### A.III Amphotere Geminitenside der allgemeinen Formel (A.III) analog WO 97/31890

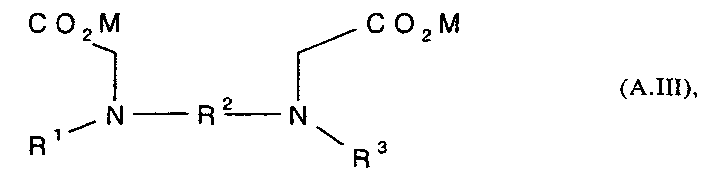

wobei die Substituenten die für die allgemeine Formel (A.I) angegebene Bedeutung haben. Geminitenside der allgemeinen Formel (A.III) sind amphotere Verbindungen, so daß sie bei entsprechend saurem Umgebungsmedium auch kationisch werden können.

### Variante B: Auf amid- oder aminhaltigen Spacern beruhende Strukturen

### B.I Geminitenside der allgemeinen Formel (B.I) analog DE 19622612 oder JP-A 10-175934

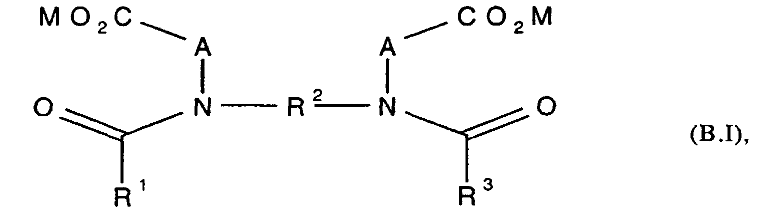

wobei die Substituenten folgende Bedeutung haben:
- R¹, R³: C₅- bis C₂₅- Alkyl, verzweigt oder unverzweigt, gesättigt, gegebenenfalls bis zu 2-fach nicht-benachbart ungesättigt;
- R²: C₁- bis C₁₂-Alkylen;
- A: CHR⁴, CH₂, C₂H₄, C₃H₆, C₄H₈;
- R⁴: Rest einer Aminocarbonsäure und
- M: Alkali, (Alkyl)Ammonium, Alkanolammonium, H oder ½ Erdalkali.

### B.II Geminitenside der allgemeinen Formel (B.II) analog EP 0 708 079

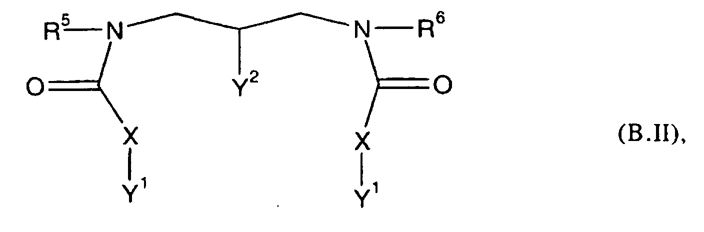

wobei die Substituenten die für die allgemeine Formel (B.I) angegebene Bedeutung haben und
- R⁵, R⁶: C₆- bis C₃₆-Alkyl, verzweigt oder unverzweigt, gesättigt, gegebenenfalls bis zu 2-fach nicht-benachbart ungesättigt;
- X: Alkylen- oder Alkenylengruppe mit 1 bis 6 Kohlenstoffatomen, die mit einer Hydroxylgruppe oder einer Sulfonsäuregruppe oder einer Carboxygruppe substituiert sein kann;
- Y¹: eine Sulfonat- oder Sulfatgruppe oder eine Carboxylgrupppe und
- Y²: eine Hydroxylgruppe, ein Schwefelsäurerest oder -O-(CO)X-COOH bedeuten.

### B.III Geminitenside der allgemeinen Formel (B.III) analog JP-A-8-311003.

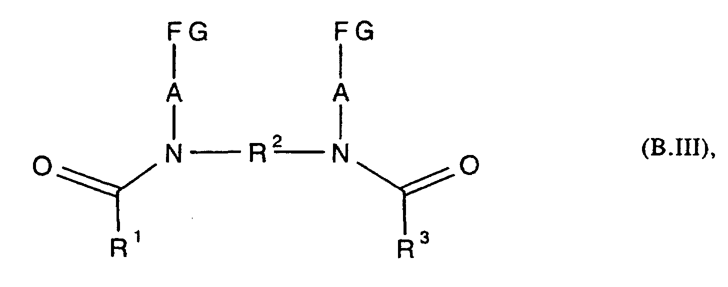

wobei die Substituenten die für die allgemeine Formel (B.I) angegebene Bedeutung haben und
- FG: -COOM oder -SO₃M bedeutet.

### B.IV Geminitenside der allgemeinen Formel (B.IV) analog JP-A 11-60437

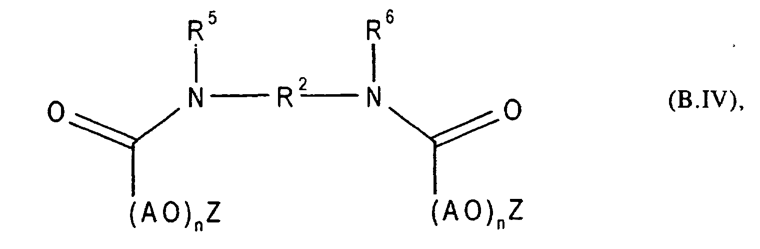

wobei die Substituenten, die bei den allgemeinen Formeln (B.I) und (B.II) angegebene Bedeutung haben und
- AO: Alkylenoxideinheiten, d.h. Ethylenglykol-, Propylenglykol und Butylenglykolethereinheiten, allein oder statistisch oder blockweise verteilt, mit n = 1 bis 20 und
- Z: -SO₃M, -C₂H₄SO₃M, -C₃H₆SO₃M, -P(O)(OM)₂ oder -CH₂-COOM, -C₂H₄-COOM bedeuten.

### Variante C: Auf amid- oder aminhaltigen Spacern beruhende Strukturen

### C.I Geminitenside der allgemeinen Formel (C.I) analog EP 0 697 244

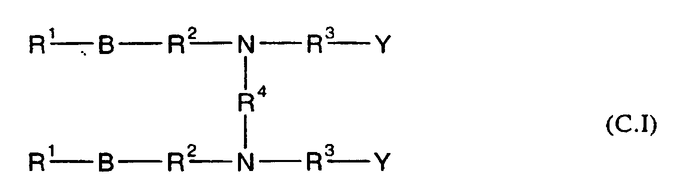

wobei die Substituenten folgende Bedeutung haben:
- R¹: C₅- bis C₂₅-Alkyl, verzweigt oder unverzweigt, gesättigt, gegebenenfalls bis zu 2-fach nicht-benachbart ungesättigt, hydroxysubstituiert oder perfluoriert ;
- R²: C₁- bis C₁₂-Alkylen oder hydroxysubstituierte Derivate davon;
- B: eine Amidgruppe [-C(O)N(R²)- oder -N(R⁵)C(O)-], eine Carboxylgruppe [-C(O)O- oder -OC(O)-], eine Polyethergruppe [-(O(R⁶-O)ₓ-];
- R⁵: für C₁- bis C₄-Alkyl oder hydroxysubstituiertes Alkyl oder H steht;
- R⁶: für C₂- bis C₄-Alkylen;
- x: eine Zahl von 1 bis 20;
- R³: für C₁- bis C₁₂- Alkyl oder hydroxysubstituierte Derivate davon, R⁷-D-R⁷ oder eine Polyethergruppe [-(O(R⁶-O)ₓ-];
- R⁷: für C₁- bis C₆- Alkylen oder hydroxysubstituierte Derivate davon;
- D: -O-, -S-, -N(R⁸)- ;
- R⁴: Alkylen oder Alkylaryl mit 1 bis 12 C-Atomen oder die hydroxysubstituierten Derivaten oder R⁹-D¹-R⁹ ;
- R⁸: C₁- bis C₁₂-Alkyl oder hydroxysubstituiertes Alkyl oder H oder R⁹-D¹-R⁹;
- R⁹: für C₁- bis C₆- Alkylen oder hydroxysubstituierte Derivate davon oder Aryl;
- D¹: -O-, .-S-, -SO₂-, -C(O)-, [-(O(R⁷-O)ₓ-], (R¹⁰)ₜ(N(R¹⁰)]_{z} oder Aryl;
- R¹⁰: C₁- bis C₁₂-Alkyl oder hydroxysubstituiertes Alkyl oder H oder Aryl;
- t,z: unabhängig voneinander eine Zahl von 1 bis 4 bedeuten und
- Y: unabhängig voneinander für -SO₃H, O-SO₃H, -OP(O)(OH)₂, -P(O)(OH)₂, -COOH, -CO₂-C₆H₄-SO₃H und deren Salze davon steht.

### C.II Geminitenside der allgemeinen Formel (C.II) analog EP 0 697 245

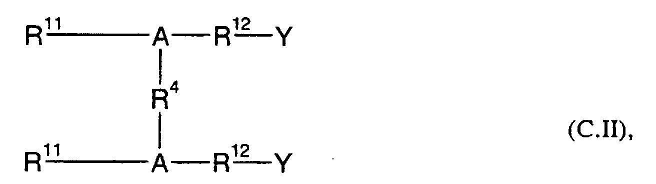

wobei die Substituenten die für die allgemeine Formel (C.I) angegebene Bedeutung haben und
- R¹¹: C₅- bis C₂₃-Alkyl, verzweigt oder unverzweigt, gesättigt, gegebenenfalls bis zu 2-fach nicht-benachbart ungesättigt, hydroxysubstituiert oder perfluoriert oder R¹⁴-B-R²;
- R¹⁴: C₁- bis C₁₂-Alkyl, verzweigt oder unverzweigt, gesättigt, gegebenenfalls bis zu 2-fach nicht-benachbart ungesättigt, oder die hydroxysubstituierten Derivate;
- R¹²: C₁- bis C₁₂-Alkylen, verzweigt oder unverzweigt, gesättigt, gegebenenfalls bis zu 2-fach nicht-benachbart ungesättigt, oder die hydroxysubstituierten Derivate oder eine Amidgruppe [-C(O)N(R²)- oder -N(R⁵)C(O)], eine Carboxylgruppe [-C(O)O- oder -OC(O)-], eine Polyethergruppe [-(O(R⁶-O)ₓ-] oder R⁹-D¹-R⁹ und
- A: -CR⁶= oder -N= unter der Voraussetzung, daß wenn A gleich -N= ist, R¹¹ gleich R¹⁴-B-R² bedeuten.

### C.III Geminitenside der allgemeinen Formel (C.III) analog DE 4227391 und DE 19608117

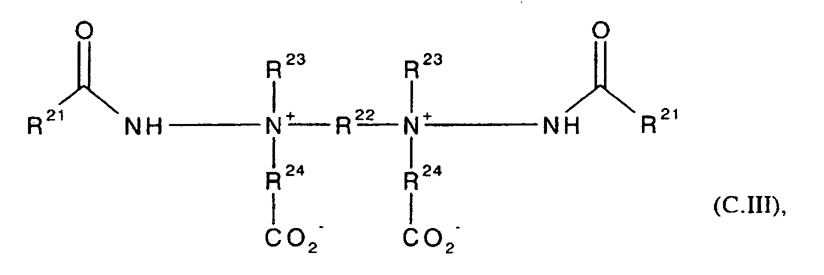

wobei die Substituenten die für die allgemeinen Formeln (C.I) und (C.II) angegebene Bedeutung haben und
- R²¹: C₅- bis C₂₃-Alkyl, verzweigt oder unverzweigt, gesättigt, gegebenenfalls bis zu 2-fach nicht-benachbart ungesättigt;
- R²², R²⁴: C₁- bis C₆ Alkylen;
- R²³: Methyl, Ethyl, Propyl oder eine Polyethergruppe [-(O(R⁶-O)ₓ-]
bedeuten.

### Variante D :

### D. I Geminitenside der allgemeinen Formel (D.I) analog US 5,863,886

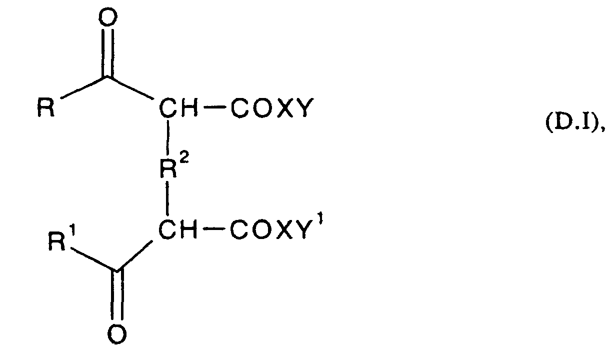

wobei die Substituenten folgende Bedeutung haben:
- R, R¹: C₅- bis C₃₀-Alkyl, verzweigt oder unverzweigt, gesättigt, gegebenenfalls bis zu 2-fach nicht-benachbart ungesättigt, hydroxysubstituiert oder perfluoriert;
- R²: C₁-bis C₁₀- Alkylen, Arylen und hydroxysubstituierte Derivate, ein Polyether [-O(R⁴O)ₓ-], -S-, -SO₂-, -O-, -S-S-, -O-R⁵-O- oder -S-R⁵-S-; Variable für eine direkte Bindung zwischen den beiden αKohlenstoffen;
- R⁴: C₂- bis C₄-Alkylen;
- R⁵: C₁- bis C₁₀-Alkylen, Arylen oder Alkylarylen, -N(R⁶)- oder -(NR⁶)-R⁷(NR⁶)- ;
- R⁶: C₁- bis C₆-Alkyl;
- R⁷: C₁- bis C₆-Alkyl, wobei R⁷ und R⁶ auch Teil eines heterocyclischen Ringes sein können;
- X: Polyether [-O(R⁴O)ₓ-], wobei x eine Zahl von 1 bis 30 ist, -O-, NZ;
- Z: C₁- bis C₁₀- Alkyl, Aryl, Alkylaryl oder H und
- Y, Y¹: unabhängig voneinander H, -CH₂-COOH und Salze, ein Kohlenwasserstoffrest mit mindestens 2 Hydroxylgruppen, wie Erythrose, Threose, Ribose, Arabinose, Xylose, Fructose, Lyxose, Allose, Altrose, Glucose, Mannose, Galactose und ihre Mischungen.

### D.II Geminitenside der allgemeinen Formel (D.II)

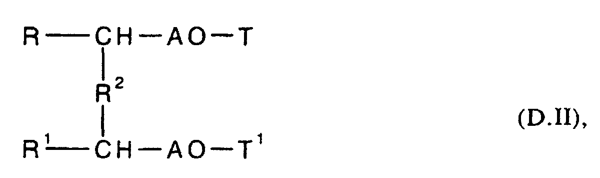

wobei die Substituenten die für die allgemeine Formel (D.I) angegebene Bedeutung haben und
- AO: -C(O)-, -C(O)- [-O(R⁴O)ₓ-], -CH₂- [-O(R⁴O)ₓ-], -CH₂-O-;
- T, T¹: unabhängig voneinander -OM, -H, -CH₃, -C₂H₅, -SO₃M, -CH₂COOM, -C₂H₄-COOM, -C₃H₆-SO₃M, -O-P(O)(OM)₂ und
- M: Alkyli, ½ Erdalkali, Ammonium, Mono-, Di-, Trialkanolammonium oder H bedeuten.

### D.III Geminitenside der allgemeinen Formel (D.III) analog WO 96/16930

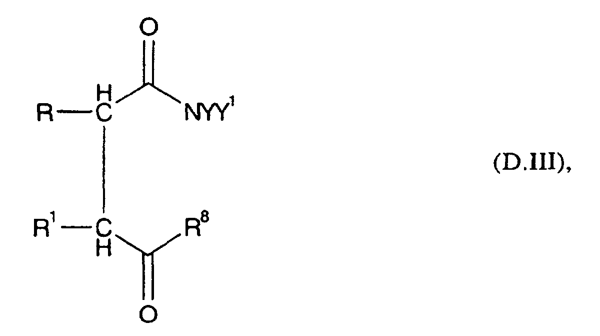

wobei die Substituenten die für die allgemeinen Formeln (D.I) und (D.II) angegebene Bedeutung haben und
- R⁸: NYY¹, -O(R⁴O)ₓH oder -O(R⁴O)ₓ-C(O)-CHR-CHR¹-C(O)NYY¹ bedeutet.

### D.IV Geminitenside der allgemeinen Formel (D.IV) analog WO 96/25384

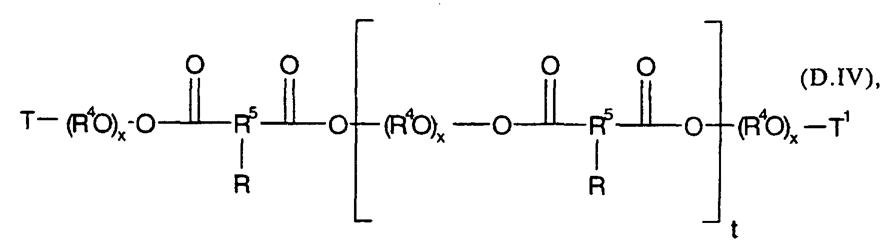

wobei die Substituenten die für die allgemeinen Formeln (D.I), (D.II) und (D.III) angegebene Bedeutung haben und
- t: eine ganze Zahl von 1 bis 100, bevorzugt 1 bis 20, besonders bevorzugt 1 bis 4 bedeutet.

Die erfindungsgemäß eingesetzten Co-Amphiphile weisen einen HLB < 6 auf, wobei sich der HLB nach der bekannten Formel HLB = E/5 (E Gew-% des hydrophilen Molekülteils) kalkulieren läßt.

Für die Verwendung der erfindungsgemäßen Tensidzusammensetzungen in Emulsionen sind besonders bei Raumtemperatur (25°) feste Co-Amphiphile geeignet, für Dispersionen sind es bevorzugt die bei Raumtemperatur flüssigen. Zu diesen Co-Amphiphilen gehören C₆- bis C₄₀-, bevorzugt C₈- bis C₂₄-Alkylalkohole, besonders bevorzugt Cetylalkohol oder Behenylalkohol, die verzweigt oder unverzweigt, gesättigt oder ein- bis dreifach nicht-benachbart ungesättigt, acyclisch oder alicyclisch sein können, nicht neutralisierte C₆- bis C₂₄-, bevorzugt C₈- bis C₂₂-Alkylcarbonsäuren, die verzweigt oder unverzweigt, gesättigt oder ein- bis dreifach nicht-benachbart ungesättigt, acyclisch oder alicyclisch sein können, Alkylarylderivate, Sorbitanester (C₆ bis C₂₂), Methylglucosidester (C₆ bis C₂₂), Zuckerester (C₆ bis C₂₂), Mono-, Di- und Triglyceride von C₆- bis C₂₂-Carbonsäuren oder Mischungen daraus, wobei Glycerin-mono-di-stearat besonders bevorzugt ist, verzweigt oder unverzweigt, gesättigt oder ein- bis dreifach nicht-benachbart ungesättigt, sowie Mono- und Diglyceride der vorgenannten Säuren, und ihre mit Milchsäure und/oder Zitronensäure weiter-veresterten Derivate, C₆bis C₂₂-Polyglycerinester, C₆- bis C₂₂-Propylenglycolester, darüber hinaus Vitaminester ( z.B. Vitamin E-acetat, Vitamin A-palmitat), Salicylsäure. Benzoesäure, Lecithine (aus Pflanzenölen oder tierischen Ursprungs). Besonders bevorzugt sind hier die Alkohole, Säuren und Mono-, Diglyceride der vorstehend genannten Carbonsäuren.

Die erfindungsgemäßen Tensidzusammensetzungen liegen mit Mischungsverhältnissen von 1 bis 70 Gew.-%, bevorzugt 5 bis 60 Gew.-% und besonders bevorzugt 5 bis 40 Gew.-% Geminitensid oder einer Mischung der geeigneten Geminitenside und entsprechend 99 bis 30 Gew.-%, bevorzugt 95 bis 40 Gew.-% und besonders bevorzugt 95 bis 60 Gew.-% Co-Amphiphil vor. Besonders bevorzugt bestehen die Tensidzusammensetzungen aus den genannten Komponenten in den genannten Anteilen. Entsprechend den genannten Mischungsanteilen von Geminitensid(e) und Co-Amphiphil können auch Mischungen meherer Co-Amphiphilen - z.B. bis zu 5 verschiedene, bevorzugt jedoch 3 verschiedene - eingesetzt werden. Besonders bevorzugt sind Mischungsanteilkomponenten aus deutlich hydrophoben und etwas hydrophileren Co-Amphiphilen. Die Verteilung ihrer Anteile richtet sich nach der Hydrophilie des Geminitensids - ist es z.B. sehr hydrophil, kann der Anteil des hydrophoberen Co-Amphiphils bei 30 bis 60 % des Co-Amphiphil-Anteiles in der erfindungsgemäßen Tensidzusammensetzung liegen, wobei die Co-Amphiphile eine HLB-Wert Differenz von größer 2 Einheiten aufweisen.

Bevorzugt sind hier Mischungen aus längerkettigen Alkoholen (ein C6- bis C40-Alkohol, aufsteigend bevorzugt C8- bis C24-, C14- bis C36- oder C14- bis C24- Alkohol), wie Cetylalkohol oder Behenylalkohol, Glycerinmono-di-stearat (GMS) und mit Zitronensäure verestertem Glycerinmonostearat oder als weitere Ausführungsform der Tensidzusammensetzung aus längerkettigen Alkoholen, wie Cetylalkhol oder Behenylalkohol oder Erucaalkohol, GMS und Stearinsäure, ganz besonders bevorzugt sind Mischungen aus Behenylalkohol, GMS und mit Zitronensäure verestertem Glycerinmonostearat.

Die bevorzugten Tensidzusammensetzungen weisen unabhängig voneinander neben
- dem Geminitensid, vorzugsweise zu 5 bis 25 Gew.%, besonders bevorzugt 10 bis 20 Gew.% , bezogen auf die Zusammensetzung Geminitensid / Co-Amphiphil(e),
zumindest 2, vorzugsweise 3, der unten genannten Co-Amphiphil-Komponenten unterschiedlicher Charakteristik auf:
(a) einen oder mehrere längerkettige Alkohole:
   ein C6- bis C40-, aufsteigend bevorzugt einen C8- bis C24-, C 14- bis C36- oder C14- bis C24- Alkohol,
(b) eine oder mehrere längerkettige Säuren:
   - ein C6- bis C24-, bevorzugt C8- bis C22-, Carbonsäure,
(c) einen oder mehrere Ester/Teilester eines Polyols mit einer oder mehreren Mono- oder Poly-Carbonsäuren:
   - ein Sorbitan(C6- bis C22-)ester,
   - ein Methylglucosid(C6- bis C22-)ester,
   - ein Zucker(C6- bis C22-)ester,
   - ein Mono-, Di- und Triglycerid einer C6- bis C22- Carbonsäure,
   - ein mit Milchsäure oder Zitronensäure verestertes Derivat der Mono- und Diglyceride einer C6- bis C22-Carbonsäure,
   - ein Polyglycerin(C6- bis C22-)ester,
   - ein Propylenglycol(C6- bis C22- )ester,
   - ein Vitaminester,
(d) sowie folgende weitere Co-Amphiphil-Komponenten:
   - Salicylsäure,
   - Benzoesäure und / oder
   - Lecithin.
      Die erfindungsgemäßen Tensidzusammensetzungen weisen nach einer weiteren Ausführungsform bevorzugt zumindest 2, vorzugsweise zumindest 3, der unten genanten Co-Amphihil Komponenten auf:

(a) zu 30 bis 50 Gew.% einen oder mehrere längerkettige Alkohole (a),
(c1) zu 30 bis 50 Gew.% ein Glycerin-Derivat wie ein Mono-, Di- und Triglycerid einer C6- bis C22- Carbonsäure, oder eine Verbindung ähnlichen HLB's, oder
(b)(c2) zu 5 bis 25 Gew %, vorzugsweise 10 bis 20 Gew.%, ein mit Milchsäure oder Zitronensäure verestertes Derivat der Mono- und Diglyceride einer C6- bis C22-Carbonsäure und/oder eine C6- bis C22-Carbonsäure,
jeweils bezogen auf die Zusammensetzung Geminitensid / Co-Amphiphile.

Vorzugsweise ist der längerkettige Alkohol zumindest eines der eingesetzten Co-Amphiphile und ein Ester eines Polyols mit einer oder mehreren Mono- oder Poly-Carbonsäuren, vorzugsweise mit 6 bis 22 Kohlenstoffatomen, die (eine) weitere eingesetzte Co-Amphiphil-Komponente. Bevorzugte Co-Amphiphile sind unabhängig voneinander in den Patentansprüchen näher bezeichnet.

Kommen z.B. 5 Co-Amphiphile zum Einsatz enthält die erfindungsgemäße Zusammensetzung neben dem Geminitensid zu 5 bis 25, bevorzugt 5 - 20 Gew.%, vorzugsweise
- Co-Amphiphil 1: langkettiger Alkohol (a),zu 20 - 50 %, bevorzugt 20 - 35 Gew.%,
- Co-Amphiphil 2: einen Ester/Teilester eines Polyols mit einer oder mehreren Mono- oder Poly-Carbonsäuren (c), insbesondere GMS oder Verbindung mit vergleichbaren HLB, zu 20 bis 50, bevorzugt 20 bis 35 Gew.%,
- Co-Amphiphil 3: zu 5 bis 25 %, bevorzugt 10 bis 20 Gew.%,
- Co-Amphiphil 4: zu 5 bis 25 %, bevorzugt 10 bis 20 Gew.% und
- Co-Amphiphil 5: zu 5 bis 25 %, bevorzugt 10 bis 20 Gew.%.

Ein besonders bevorzuge Ausführungsform der Erfindung weist folgende Zusammensetzung auf:

| | | |
|---|---|---|
| Geminitensid | 5 bis 15 | Gew.%, |
| Glycerinmono-distearat | 30 bis 40 | Gew.%, |
| Behenylalkohol | 35 bis 45 | Gew.% und |
| Glycerylstearatcitrat | 10 bis 20 | Gew. %. |

Das hierbei eingesetzte Geminitensid ist besonders bevorzugt ein Geminitensid von Typ A.

Ohne die Erfindung auf diesen Mechanismus einzuschränken, kann man annehmen, daß die erfindungsgemäßen Tensidzusammensetzungen eine überraschend hohe Neigung zur Ausbildung ausgeprägter flüssigkristalliner lamellarer Phasen oder auch zu vesikulären Strukturen haben, die eine ganz erstaunliche Grenzflächenelastizität aufweisen und so hoch effizient zu stabilen feinteiligen Emulsionen oder sehr stabilen Dispersionen führen. Die flüssigkristallinen lamellaren Phasen, die man mit Hilfe der erfindungsgemäßen Tensidzusammensetzungen erhält, haben eine besonders weite Ausdehnung im Phasendiagramm der Anwendungsformulierung. Die flüssigkristallinen lamellaren Phasen können als Gelnetzwerk die jeweiligen Formulierungen stabilisieren und/oder können als viskositätsgebende dritte Phase zur Steuerung des jeweiligen Systems hinsichtlich seiner Viskosität aber auch hinsichtlich der Spreitung eingesetzt werden.

Die erfindungsgemäße Tensidzusammensetzung bildet in einer O/W-Emulsion, d.h. bei Anwesenheit von Öl und Wasser in abhängig vom Mischungsverhältnis, eine dritte Phase. Die dritte Phase, ein dreidimensionales Netzwerk, stabilisiert einerseits die Trennung von Öl und Wasser (die Wasserhärte spielt bei Verwendung anionischer Geminitenside hier nur noch eine untergeordnete Rolle) und erhöht andererseits die Viskosität der Mischung. Das wiederum führt zu einer weiteren Stabilisierung. "Creaming" beispielsweise wird dadurch stark verlangsamt oder bei einer optimalen Formulierung völlig verhindert. Wichtig für die Stabilität der durch ein Gelnetzwerk stabilisierten Emulsion ist der Erweichungspunkt des Gelnetzwerkes, der sehr stark vom Schmelzpunkt des als Co-Amphiphil verwendeten Alkohols beeinflußt wird - darum werden hier nur langkettige Alkohole eingesetzt.

Überraschend hat sich bei den erfindungsgemäßen Tensidzusammensetzungen gezeigt, daß ein und dieselbe Kombination als sehr guter Emulgator mit einer großen Bandbreite hinsichtlich der Polarität des verwendeten Öls oder Ölmischungen (inklusive Siliconöl) wie auch als Dispergator mit einer sehr erstaunlichen Bandbreite hinsichtlich der Oberfläche des dispergierten Pigmentes (hydrophil oder hydrophob beschichtet) als auch hinsichtlich des Mediums (in Öl oder in Wasser dispergiert) geeignet ist. Die Fachwelt hat bisher die These vertreten, daß jede Aufgabe, d.h. z.B. Dispersion eines hydrophilen Pigmentes in Wasser gegenüber der Dispersion eines hydrophilen Tensids in Öl, seine individuelle Lösung, also seine individuelle Dispergator- oder auch Emulgatorkombination (wiederum Geminitensid + Co-Amphiphil(e)) verlangt.

Weiterhin hat sich überraschend herausgestellt, daß die erfindungsgemäßen Tensidzusammensetzungen eine sehr hohe pH- wie auch Elektrolyttoleranz aufweisen und zu einer Reduktion der Lipidperoxidkonzentration durch Verwendung der Mischung in einer Creme oder Lotion führen. Kombinationen mit anionischen Tensiden galten bisher als sehr empfindlich gegenüber Elektrolyten in Formulierungen.

### Herstellungsverfahren der Tensidzusammensetzungen

Ein besonders bevorzugtes Verfahren zur Herstellung der erfindungsgemäßen Tensidzusammensetzungen in Form von Emulsionen mit außerordentlich geringer Tröpfchengröße (z.B. kleiner 1 µm) der diskontinuierlichen Phase ist die Phasentransfer-Temperatur-Methode (PTT, Phase Transfer Temperature). Die PTT Methode lehnt sich an die für ethoxylierte Tenside beschriebene Phaseninvertmethode (K. Shinoda, H. Saito, J. Colloid Interface Sci., 34 (1969) 238, welche hiermit durch Referenz zum Inhalt dieser Schrift gemacht wird) insofern an, als daß auch sie einen Phasenübergang mit einer extrem niedrigen Grenzflächenspannung ausnutzt. Der Phasenübergang wird definiert durch den Übergang von einer mizellengeprägten Phase zu einer überwiegend lamellaren Phase.

Das Co-Amphiphil wird kurz oberhalb einer kritischen Temperatur, die durch seinen Schmelzpunkt und seine Löslichkeit in der Ölphase festgelegt ist, mit der wäßrigen Geminitensid enthaltenden Phase, die sich knapp unterhalb dieser Temperatur befindet, kombiniert. Bei der Mischung der beiden Phasen wird wahrscheinlich das Co-Amphiphil - beschleunigt durch die durch Abkühlung schlechter werdende Löslichkeit in der Ölphase - verstärkt in die Mizellen der Geminitenside aufgenommen. Bei der hohen Neigung der nun vorhandenen Mischung, eine flüssigkristalline lamellare Phase auszubilden, entsteht diese, begünstigt durch das rasche Anwachsen der Mizellen, sehr schnell und führt zu überraschend kleinen und stabilen Öltröpfchen, welche erfindungsgemäß bevorzugt Tröpfchendurchmesser von kleiner 1 µm aufweisen

Das so erhaltene Ergebnis für die erfindungsgemäßen Tensidzusammensetzungen ist signifikant besser als das Vorgehen nach den klassischen Emulgiermethoden (Übersicht J. Britto, Euro Cosmetics, 7-8 (1998) 30). Setzt man umgekehrt die PTT Methode für Mischungen aus konventionellen Tensiden und Co-Amphiphilen ein, verbessern sich deren Eigenschaften im allgemeinen nicht.

### Anwendungsbeispiele

Die nachfolgend aufgeführten Verwendungen zeigen beispielhaft die möglichen Anwendungsfelder auf. Besonders bevorzugt ist der Einsatz der erfindungsgemäßen Tensidzusammensetzungen als Emulgier- oder Dispergierhilfsmittel, als Zusatz zu konventionellen (Nicht-Gemini-)anionischen Tensiden oder als Zusatz zu Haarund Hautreinigungsmittel-Formulierungen.

Die erfindungsgemäßen Tensidzusammensetzungen können zur Formulierung von O/W-, W/O- Emulsionen (z.B. unter Einsatz von Lecithin als Co-Amphiphil) und von Microemulsionen eingesetzt werden.

Die erfindungsgemäßen Tensidzusammensetzungen sind in Anwendungsformulierungen in der Kosmetik, Körperpflege und Dermatologie, in der Agrochemie wie auch zur Formulierung von Beschichtungsmitteln wie Lacken und Farben (als Dispergatorsystem, als Agens im Primer, als Additiv zur Verbesserung der Dispersion und Tröpfchengrößenverteilung der organischen Phase in wasserbasierenden Lacken) oder als Tinten und Druckfarben einsetzbar. Schließlich eignen sich die erfindungsgemäßen Tensidzusammensetzungen auch in ihrer Eigenschaft als Emulgatoren und Dispergatoren zur Vereinfachung oder Verbesserung der Applikationsformen von Pharmaka, z.B. zur kontrollierten Wirkstoff-Freigabe "(controlled release") von Pharmaka.

Die vorliegende Erfindung betrifft damit als besondere Ausführungsform kosmetische und dermatologische Zubereitungsformen wie Reinigungsemulsionen, Gesichts- und Körperpflegezubereitungen, haarkosmetische Zubereitungen zur Pflege des Haares und der Kopfhaut, Zubereitungen für die Mund- und Gebißhygiene und -pflege, kosmetische und dermatologische Lichtschutzzubereitungen und kosmetische Desodorantien.

Die erfindungsgemäßen Tensidzusammensetzungen können in nachfolgende Formulierungen für die Körperpflege oder kosmetische oder dermatologische Zwecke eingesetzt werden: in Pumpsprays, Aerosolsprays, Cremes, Salben, Tinkturen, Lotionen, Nagelpflegeprodukten (z.B. Nagellack, Nagellackentferner, Nagelbalsame), Bodylotionen, Aftershaves, Hautaufhellungsmittel, Bräunungscremes, wasserfeste Sonnenschutzcremes oder -lotionen, dekorative Kosmetika (wie Lippenstifte und Eyeliner); Shampoos, Antischuppenshampoos, Haarkuren, Haarfestiger, Haarspülungen, Babyshampoos, Haar-Conditioner, Dauerwellformulierungen, Haar-Relaxer-Formulierungen (sogenannte Relaxer Kits zur Entfernung von Locken), Waschgele, Dusch- und Badegele bzw. Dusch- und Badezusätze, Handwaschlotionen, Desodorantien (wie Roll-on, Stift, Spray), Zahnputzmittel, Gebißreiniger, Mundwässer, Schaumbäder, Ölbäder, Ölschaumbäder, Make-up-Entferner besonders Augenmake-up-Entferner, Gesichtsreinigungscremes, Haarcremes (Pomaden), Feuchtigkeitscremes, Pflegecremes sowie Tagescremes (mit oder ohne zusätzlichen Lichtschutz), Fußcremes, liposomhaltige Gele, Haarconditioniergele, Haarentfernungsmittel (z.B. in Form von Cremes), Rasiergele oder -schäume, Massagecremes, Pflegemasken, Foundationcremes, Haarwellmittel, Haarfärbemittel, Stückseifen vom Kombibar-Typ, Syndetseifen und flüssige Handwaschseifen.

Von den sonstigen Ingredienzien, mit denen die erfindungsgemäßen Tensidzusammensetzungen bei der Herstellung von Formulierungen für die Körperpflege oder kosmetische Zwecke kombiniert werden können, seien beispielhaft genannt: Alkylsarcosinate, Cellulose- und Guarderivate, Aromaöle, Lavendel-, Anis-, Rosmarin-, Fichtennadel- und Latschenkiefernöl, Teebaumöl oder Calendula-Öl, Nachtkerzenöl, Mundpflegearomaöle (z.B. "Dragoco ZM 0065"), Parfümöle; Pflegeöle wie A-vocado-, Jojobaöl oder Aloe Vera; dialkylierte Essigsäure, UV-Absorber (wie in der EU-Direktive Nr 76/768/CEE und ihren Anhängen und Modifikationen aufgeführt), Dihydroxyaceton, Benzophenon, Octyltriazon, Methoxyzimtsäure und ihre Derivate, Melanin und -Derivate, langkettige Dialkylether, Methylbenzylidencampher, Ester der Salicylsäure, Hyaluronsäure und ihre Derivate, Cyclodextrine (leer z.B. als Geruchshemmer oder gefüllt z.B. mit Duftstoffen und/oder Wirkstoffen), Vitamine wie Vitamin A oder E, Vitaminderivate wie Vitamin A-Palmitat, Squalan, Squalen, β-Carotin und weitere Farbstoffe, Tocopherol und Tocopherolderivate (wie Tocopherolacetat), Retinylpalmitat, Bisabolol, d-Panthenol, Ascorbinsäure, Antioxydantien, Pflanzensteroide (wie Ergosterin und β-Sitosterin) und deren Derivate, Cholesterin und dessen Derivate, Parabene und deren Derivate (wie Methyl-, Ethyl-, Propyl- und Butyl-Paraben), Perleffektstoffe, Entzündungshemmer, Ceramide, Pseudoceramide, Imidazolidinylharnstoff, Diisoarachidyldilinoleat, Polymere (wie Polyacrylamide, Carboxyvinyl-Polymere, Maleinsäureanhydrid-Oleat-Copolymere, Polyethylenglykolmono- oder -diester, Polyvinylpyrrolidon, Polysaccharide, Polyacrylate, Fluorkohlenwasserstoffe), kationische Polymere (wie Diethyldiallylammoniumchlorid-Acrylamid-Copolymere, Antitranspirantien (wie Aluminium- oder Zirkoniumsalze), Zitronensäure, Milchsäure, Octylmethoxycinnamat, Phospholipide, Natriumpyrrolidoncarboxylat, Gelatine, Alginate, Albumin, Kollagen und Kollagenderivate, Bienenwachs, Wachsester aus langkettigen Carbonsäuren (verzweigt oder unverzweigt) und langkettigen Alkoholen (verzweigt oder unverzweigt), Dimethylsiloxane (acyclisch, cyclisch, leicht flüchtig bis ölartig), Phenyltrimethicon, Xanthangummi, Stärkederivate, Glycerin, Ascorbinsäure, Polyethylenglykole sowie ihre Mono- und Dicarbonsäureester, Fettsäuremono-, -di- und -triglyceride und ihre Derivate (- sulfate, -citrate, -lactylate, -lactate, -tartrate), Carnaubawachs, Lecithin, Chlorhexidin-Salze, Benzethoniumchlorid, Benzalkoniumchlorid, Triclosan, Triclocarban, Methylchlorisothiazolin, Methylisothiazolin, Chlorxylenol, DMDM-Hydantoin, Alkyltrimethylammoniumbromid, Salicylsäure und deren Derivate, Inosit-Derivate, acylierte Ethylendiamin-Derivate, für kosmetische Zwecke zugelassene Farbstoffe (wie sie beispielsweise in der Veröffentlichung "Kosmetische Färbemittel" der Farbstoffkommission der Deutschen Forschungsgemeinschaft, Verlag Chemie. Weinheim, 1984, Seite 81 ff., zusammengestellt sind) sowie Alkohole von C₆ bis C₂₄, Guerbetalkohole und -säuren.

Weitere Ingredienzen sind:
Antioxidantien , z.B. gewählt aus der Gruppe bestehend aus Aminosäuren (z. B. Glycin, Histidin, Tyrosin, Tryptophan) und deren Derivaten, Imidazole (z. B. Urocaninsäure) und deren Derivate, Peptide wie DL-Carnosin, D-Carnosin, L-Carnosin und deren Derivate (z. B. "Anserin X") Carotinoide, Carotine (z. B. α-Carotin, β-Carotin, Lycopin) und deren Derivate, Liponsäure und deren Derivate (z. B. Dihydroliponsäure), Aurothioglucose, und andere Thiole (z. B. Thioredoxin, Glutathion, Cystein, Cystin, Cyftamin und deren Glycosyl-. N-Acetyl-, Methyl-, Ethyl-, Propyl-, Amyl-, Butyl- und Lauryl-, Palmitoyl-, Oleyl-, y-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen, Homocysteinsulfoxiniin, Buthioninsulfone, Penta-, Hexa-, Heptathioninsulfoxiniin) in sehr geringen verträglichen Dosierungen ferner (Metall-)Chelatoren (z. B. α-Hydroxyfettsäuren, Palmitinsäuren, Phytinsäuren, Lactoferrin-, -Hydroxysäuren (z. B. Zitronensäure, Milchsäure, Apfelsäure, Huminsäure, Gallensäure), Gallenextrakt Biliräbin, Biliverdin, EDTA, und deren Derivate, ungesättigte Fettsäuren und deren Derivate (z. B. γ-Linolensäure, Linolsäure, Olsäure, Folsäure und deren Derivate), Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin E-acetat), Vitamin A und Derivate (Vitamin A-palmitat) sowie Konylkrylbenzoat des Benzoeharzes, Rufinsäure und deren Derivate, Ferulasäure und deren Derivate, Butylhydroxytoluol, Butylhydroxyanisol, Nordihydroguajakharnsäure, Nordihydroguajaretsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die Derivate (Salze, Ester, Ether, Zucker, Nukleotide, Nukleoside, Peptide und Lipide) dieser genannten Wirkstoffe. Die Menge der Antioxidantien (eine oder mehrere Verbindungen) in den Zubereitungen liegt vorzugsweise bei 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen. Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.%. bezogen auf das Gesamtgewicht der Formulierung, zu wählen.

### Weitere Ingredienzen sind:

UVB-Filter (öllöslich oder wasserlöslich), als öllösliche Substanzen sind z. B. zu nennen: 3-Benzylidencampher und dessen Derivate, z.B, 3,4,4-Trimethylbenzyliden-campher, 4-Aminobenzoesäure-Derivate, vorzugsweise 4-Dimethylamino-benzoesäure(2-ethylhexyl)ester, 4-Dimethylamino-benzoesäureamylester, Ester der Zimtsäure, vorzugsweise 4-Methoxyzimtsäure(2-ethyIhexyl)ester, 4-Methoxyzimtsäureisopentylester; Ester der Salicylsäure, vorzugsweise Salicylsäure-(2-ethylhexyl)ester, Salicylsäure-(4-isopropylbenzyl)ester, Salicylsäurehomomenthylester; Derivate des Benzophenons, vorzugsweise 2-Hydroxy-4-methoxybenzophenon, 2-Hydroxy-4-methoxy-methylbenzophenon, 2,2'-Dihydroxy-4-methoxybenzophenon; Ester der Benzylmalonsäure; als wasserlösliche Substanzen sind vorteilhaft: 2-Phenylbenzimidazol-5-sulfonsäure und deren Salze, z. B. Natrium-, Kalium- oder Triethanolammonium-Salze, Sulfonsäure-Derivate von Benzophenonen und ihre Salze, Sulfonsäure-Derivate des 3-Benzylidencamphers und ihre Salze.

UVA-Filter, die üblicherweise in kosmetischen und/oder dermatologischen Zubereitungen enthalten sind. Bei solchen Substanzen handelt es sich vorzugsweise um Derivate des Dibenzoylethans oder anorganische Pigmente (bevorzugt ZnO).

Üblicherweise werden in der Kosmetik zum Schutze der Haut vor UV-Strahlen (UVA und UVB) auch anorganische Pigmente eingesetzt, dabei handelt es sich um Oxide des Titans, Zinks, Eisens, Zirkoniums, Siliciums, Mangans, Aluminiums, Cers und Mischungen davon, sowie Abwandlungen oder Kombinationen (z.B. Ti- und Fe-Oxide), bei denen die Oxide die aktiven Agentien sind. Die vorgenannten Metalloxide sind im allgemeinen entweder mit einer hydrophilen (z.B. Glycerin) oder mit einer hydrophoben Beschichtung, beispielsweise Alkylsilane oder Metallseifen versehen.

Die erfindungsgemäßen Tensidzusammensetzungen eignen sich weiterhin als Zusatzstoffe - beispielsweise als Emulgatorsystem oder als Additiv, um Applikationsparameter anzupassen - zu Pflanzenschutzmitteln, insbesondere Fungiziden, Herbiziden (z.B. mit Wirkstoffen wie Glyphosaten oder Sulfosaten), Insektiziden, Nematociden, Acariciden und Wachstumsregulatoren. Sie emulgieren die Wirkstoffe zu stabilen Spritzbrühen, die die besprühten oder begossenen Objekte gut benetzen und die Wirkstoffe, falls erforderlich auch kontrolliert, über einen längeren Zeitraum freisetzen.

Die erfindungsgemäßen Tensidzusammensetzungen können weiterhin z.B. in Abkochhilfsmitteln für Baumwolle (Beuchen oder Brühen), der Rohwollwäsche, in Walkhilfsmitteln, Egalisierhilfmitteln, Schmelz- und Präparationsmitteln, Reservierungsmitteln, Avivagen, Dispersionen, Antielektrostatika, Detachiermitteln, Tierhautentfettungsmitteln, Gerbmitteln und Lederfettungsmitteln angewandt werden.

Auch in Desinfektionsmitteln können die erfindungsgemäßen Tensidzusammensetzungen zusammen mit allen bekannten desinfizierenden Stoffen eingesetzt werden. Solche desinfizierenden Stoffe sind z. B. Phenole, Kresole, Chlorhexidinsalze, Benzethoniumchlorid, Benzalkoniumchlorid, Triclosan, Triclocarban, Methylchlorisothiazolidin, Chlorxylenol, DMDM-Hydantoin und Alkyltrimethylammoniumbromid.

Die erfindungsgemäßen Tensidzusammensetzungen eignen sich auch als Dispergatorsystem in Beschichtungsmitteln. Beispielsweise dispergieren und stabilisieren sie wirkungsvoll Pigmente in wasserverdünnbaren Lacken. Zu den dispergierbaren anorganischen Pigmenten zählen Titanoxid, Eisenoxid, Ceroxid, Aluminumoxid, Calciumcarbonat, Calciumphosphat, Talcumpulver, Kaolin, Bariumsulfat, Aluminium- und Zirkonsalze. Zinkoxid, Silikate und Alumosilikate. Von den organischen Pigmenten seien beispielhaft Phthalocyanin-Grün und -Blau, Ruße und Graphit genannt. Ebenso können die erfindungsgemäßen Tensidzusammensetzungen in Dispersionsfarben eingesetzt werden, wo sie wiederum Pigmente, aber auch die polymeren Bindemittelteilchen dispergieren, die Dispersion stabilisieren und das Benetzen der Substrate fördern, beispielsweise bei ihrem Einsatz in Vorstrichmitteln und Primern. Die erfindungsgemäßen Tensidzusammensetzungen eignen sich ebenfalls als Dispergatoren und / oder Stabilisatoren in Tinten und Druckfarben.

Weiterhin lassen sich die erfindungsgemäßen Tensidzusammensetzungen als Dispergatorsystem in therapeutischen Zubereitungen verwenden. Diese Verwendung ähnelt der in Kosmetika. Ebenso wie in Cremes, Salben, Lotionen usw. mit kosmetischen Wirkstoffen können die Tensidzusammensetzungen auch in Cremes, Salben, Lotionen usw. mit therapeutischer Zielrichtung eingesetzt werden.

Eine andere Anwendung finden die erfindungsgemäßen Tensidzusammensetzungen bei der Emulsions- oder Suspensionspolymerisation, beispielsweise zur Herstellung von (Meth)acrylat-, Vinylacetat- oder Vinylpropionatdispersionen für Anstrich- oder Klebezwecke oder von (Co)polymerdispersionen von Acrylamid, Acrylsäure, Acrylsäureestern, Acrylnitril, Maleinsäureanhydrid, Styrol und/oder Butadien, die durch radikalisch initiierte Polymerisation, z. B. mit Azoisobutyronitril als Starter, hergestellt wurden.

Die erfindungsgemäßen Tensidzusammensetzungen können bereits vor einem Einsatz auf einem der vorstehend aufgeführten Anwendungsgebiete mit anderen Komponenten zusätzlich gemischt werden, dann besteht die Tensidzusammensetzung nicht mehr nur aus den beiden zwingenden Komponenten, sondern sie enthält zusätzlich zu diesen weitere Komponenten. Das Einmischen der zusätzlichen Komponenten kann vor dem Einsatz oder auch gleichzeitig mit dem Einsatz auf einem der Anwendungsgebiete erfolgen. Dazu zählen Tenside aber auch andersartige Komponenten, die für den jeweiligen Verwendungszweck der Formulierungen, Mittel, Mischungen, Zubereitungen usw. als Zusatzstoffe bekannt sind. Solche Zusatzstoffe werden in der Folge aufgezählt. Dem Fachmann ist geläufig, welcher Zusatzstoff für welche Verwendung bzw. Formulierung einsetzbar ist. Als Zusatzstoffe seien insbesondere Enzyme, Enzymstabilisatoren, Bleichsysteme, Chelat-bildende Agentien, optische Aufheller und Schauminhibitoren genannt.

Neben den Geminitensiden können jeweils Kombinationen oder einzelne der im folgenden genannten Tenside für die erfindungsgemäßen Verwendungen mit den Geminitensiden kombiniert werden. Hierbei werden gegebenenfalls 0,1 bis 99,9 Gew.-% dieser Tenside eingesetzt, bezogen auf das Gesamtgewicht der verschiedenen Tenside. Als nicht limitierende Beispiele für nichtionische grenzflächenaktive Substanzen seien Fettsäureglyceride, Fettsäurepolyglyceride, Fettsäureester, Alkoxylate höherer Alkohole, alkoxylierte Fettsäureglyceride, Polyoxyethylenoxypropylenglykolfettsäureester, Polyoxyethylensorbitanfettsäureester, Polyoxyethylen-Rhizinusöl- oder gehärtete Rhizinusölderivate, Polyoxyethylenlanolinderivate, Polyoxyethylenfettsäureamide, Polyoxyethylenalkylamine, Derivate von Alkanolaminen, Alkylaminoxide, Derivate von Eiweißhydrolysaten, Hydroxymischether, Alkylmono- oder -poly-glycoside und Alkylglucamide (z.B. N-Methylalkylglucamide) aufgeführt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, α-Sulfofettsäurederivate (einschließlich der in VVO 93/25646 beschriebenen), Dicarbonate (wie in DE-A-196 22 612 beschrieben), Sulfonate höherer Fettsäureester, höhere Alkoholsulfate (primär und sekundär), Alkoholethersulfate, Hydroxymischethersulfate, Sulfate und Carbonate von alkoxylierten Carbonsäurealkanolamiden, Salze von Phosphatestern, Tauride, Isethionate lineare Alkylbenzolsulfonate, verbrückte Alkylbenzolsulfonate, d.h. Dialkyl-mono- oder -disulfonate des Diphenylethers, Alkylarylsulfonate, Sulfate der Polyoxyethylenfettsäureamide und Derivate von Acylaminosäuren, Alkylethercarbonsäuren, Alkyl- und Dialkylsulfosuccinate, Alkenylsulfosuccinate, Alkyl- oder Alkenylsarcosinate und sulfatierte Glycerinalkylether genannt.

Als Beispiele für kationische grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Imidazoliniumderivate, Alkylpyridiniumsalze, quaternierte Fettsäureester von Alkanolaminen, Alkylisochinoliniumsalze, Benzethoniumchloride und kationische Acylaminosäurederivate genannt.

Als Beispiele für ampholytische und betainische grenzflächenaktive Substanzen, die für Kombinationen eingesetzt werden können, seien Carbobetaine wie Kokosacylamidopropyldimethylbetain, Acylamidopentandiethylbetain, Dimethylammonio-hexanoat-acylamidopropan-(oder -ethan-)dimethyl-(oder-diethyl-)-betain, alle mit C- Kettenlängen zwischen 10 und 18 , Sulfobetaine, Imidazolinderivate, Sojaöllipide und Lecithin genannt. Die erwähnten Amin-N-oxide können auch in polymerer Form vorliegen, wobei ein Verhältnis Amin zu Amin-N-oxid von 10:1 bis 1:1 000 000 vorliegen muß, die mittlere Molmasse beträgt 500 bis 1 000 000, besonders bevorzugt jedoch 5 000 bis 100 000.

Verschiedene weitere Komponenten können jeweils einzeln oder in Kombinationen mit den erfindungsgemäßen Tensidzusammensetzungen kombiniert werden. Von diesen Komponenten seien genannt: Trägerstoffe, Hydrotropica, Prozesshilfsmittel, Farbstoffe oder Pigmente, Parfums, Lösungsmittel für Flüssigformulierungen (besonders bevorzugt sind Alkohole mit 1 bis 6 Kohlenstoffatomen und 1 bis 6 Hydroxygruppen), feste Füller für Stückseifenformulierungen, Perlglanzmittel, (z. B. Distearoylglyceride), Konservierungsmittel oder Pufferungssysteme. Sollte ein höheres Schäumvermögen der Formulierung, wie in einigen Körperpflegemitteln, erforderlich sein, so kann dieses z. B. durch den Zusatz von C₁₀- bis C₁₆- Alkanolamiden (in Konzentrationen von 1 bis 10 Gew.-% der Gesamtformulierung) erhöht werden. Auch weitere wasserlösliche Magnesiumsalze können zur Erhöhung des Schäumvermögens und der Fettlösekraft in Mengen von 0,1 bis 2 Gew.-% zugesetzt werden. Wenn notwendig, können einige der obengenannten Tensidkomponenten auch durch Adsorption auf poröse hydrophobe Substanzen stabilisiert und mit einer weiteren hydrophoben Schicht versiegelt in die Formulierung eingearbeitet werden.

### Beispiele

Die folgenden Beispiele sollen verschiedene Ausführungsformen der vorliegenden Erfindung näher erläutern.

### Herstellung von Dispersionen:

Als Micropigmente werden ein mit Aluminium und Glycerin gecoatetes Titandioxid des Typs UV Titan M 212 (Hersteller Kemira, Finnland) und ein mit Dimethicone (INCI-Nomenklatur) gecoatetes Zinkoxid des Typs Z-Cote HP 1 (Hersteller Sun Smart, USA) eingesetzt. Zum Vergleich werden handelsübliche Dispersionen eingesetzt und vermessen.
- Dispersion in Wasser:: "Tioveil AQ", (Hersteller Tioxide), ca. 40 Gew.-% Titandioxid in Wasser dispergiert.
- Dispersion in Öl:: "Tioveil MIG", (Hersteller Tioxide), ca. 40 Gew% Titandioxid in Caprin-/Capryl- Triglycerid dispergiert (z.B. "Miglyol 812" der Condea Chemie GmbH).

Die Bestimmung der Partikelgrößenverteilung erfolgt mit Hilfe des Laser-Light-Scattering Verfahrens. Geminitensid-Mischungen der anderen erfindungsgemäßen Tensidzusammensetzungen zeigen ein ähnliches Verhalten. Für diese wird lediglich die Größe Gew.-% Partikel < 1 µm tabellarisch aufgeführt.

| **Geminitensid (Formel)** | **Variante A Struktur** |
|---|---|
| A.A (A.I) | R¹ = R³ = C₁₁H₂₃/C₁₃H₂₇, R² = C₂H₄, X = Y = (C₂H₄O-)ₓ(C₃H₆O-)_{y}SO₃Na mit x = 14, y = 0 |
| A.B (A.I) | R¹ = R³ = C₁₁H₂₃/C₁₃H₂₇, R² = C₂H₄, X = Y = (C₂H₄O-)ₓ(C₃H₆O-)_{y}H mit x = 14, y = 0 |
| A.C (A.I) | R¹ = R³ = C₁₁H₂₃/C₁₃H₂₇, R² = C₂H₄, X = Y = (C₂H₄O-)ₓ(C₃H₆O-)_{y}SO₃TIPA mit x = 11, y = 0; TIPA = Triisopropanolammonium |
| A.D (A.I) | R¹ = R³ = C₁₁H₂₃/C₁₃H₂₇, R² = C₂H₄, X = Y = (C₂H₄O-)ₓ(C₃H₆O-)_{y}SO₃Na mit x = 11, y = 0 |
| A.E (A.I) | R¹ = R³ = C₁₁H₂₃/C₁₃H₂₇, R² = C₂H₄, X = Y = (C₂H₄O-)ₓ(C₃H₆O)_{y}CH₂CO₂Na mit x = 14, y = 0 |

| **Geminitensid (Formel)** | **Variante B Struktur** |
|---|---|
| B.A (B.I) | R¹ = R³ = C₁₁H₂₃/C₁₃H₂₇, R² = C₂H₄, A = CH₂, M = Na |
| B.B (B.II) | R⁵ = R⁶ = C₁₂H₂₅/C₁₄H₂₉, X = C₂H₄, Y¹ = CO₂Na, Y² = -O-C(O)-C₂H₄-CO₂Na |
| B.C (B.II) | R⁵ = R⁶ = C₁₂H₂₅/C₁₄H₂₉, X = C₂H₄, Y¹ = CO₂Na, Y² = -O-C(O)-C₂H₄-CO₂Na |
| B.D (B.III) | R¹ = R³ = C₁₁H₂₃/C₁₃H₂₇, R² = C₂H₄, A= C₂H₄, FG = -SO₃Na |

| **Geminitensid (Formel)** | **Variante C Struktur** |
|---|---|
| C.A (C.I) | R¹ = C₁₁H₂₃, B = C₂H₄, R³ = CH_{2,} R⁴ = C₂H₄, Y = COONa |
| C.B (C.I) | R¹ = C₁₄H₂₉, B = C₂H₄, R³ = C₂H_{4,} R⁴ = C₂H₄, Y = COONa |
| C.C (C.II) | R¹¹ = C₁₂H₂₅-C(O)-C₂H₄- , A = N, R¹² = C₂H_{4,} R⁴ = C₂H₄, Y = OH |
| C.D (C.V) | R²¹ = C₁₁H₂₃/C₁₃H₂₇, R²², R²³, R²⁴ = C₂H₄ |

| **Geminitensid (Formel)** | **Variante D Struktur** |
|---|---|
| D.A (D.I) | R, R¹ = -C₁₁H₂₃, R² = -S-, X = NZ, Z = -CH₃, Y, Y¹ = Glucosylrest |
| D.B (D.II) | R, R¹ = -C₁₁H₂₃, R² = Einfachbindung, AO = -C(O)-, T, T¹= OM, M = Na |
| D.C (D.III) | R, R¹ = C₁₂H₂₄, R⁸ = NYY1, Y = -CH₃, Y¹ = Glucosylrest |
| D.D (D.IV) | R⁵ = -C₂H₃=, R = C₁₂H₂₄, R⁴ = C₂H₄, x = 3, T, T¹ = H |

### Beispiel 1 (nicht erfindungsgemäß)

Herstellung von Pigment-Dispersionen in Wasser (Ansatzgröße 100 g): Das Geminitensid und das Co-Amphiphil wurden in Wasser bei 80 °C aufgelöst, bis eine homogene leicht trübe Dispersion entstand. Nach Abkühlen der wäßrigen Tensidphase auf ca. 30 °C wurde unter Rühren das Pigment in die Wasserphase eindispergiert. Anschließend wurde die Dispersion mit einem Ultra-Turrax-Gerät 5 Minuten bei maximaler Umfangsgeschwindigkeit homogenisiert.

| **Inhaltsstoffe** | **Gew.-% (m/m)** |
|---|---|
| Geminitensid | 4,25 |
| C₈ bis C₁₀-Fettalkohol | 0,75 |
| Wasser | 45,00 |
| Pigment | 50,00 |

Nach 24 h bei Raumtemperatur wurde eine Messung der Partikelgrößenverteilung durchgeführt.

| **Pigment** | **Geminitensid** | **Median / Partikelgrößenverteilung (µm)** |
|---|---|---|
| Titandioxid UV Titan M212 | A.A | 0,37 |
| (Hersteller Kemira) | A.B | 0,33 |
| | A.C | 0,38 |
| | A.D | 0,37 |
| | A.E | 0,36 |
| Zinkoxid Z-Cote HP 1 | A.A | 0,37 |
| (Hersteller Sun Smart) | A.B | 0,37 |
| | A.C | 0,38 |
| | A.D | 0,37 |
| | A.E | 0,39 |
| Titandioxid UV Titan M212 | B.A | 0,38 |
| (Hersteller Kemira) | B.B | 0,33 |
| | C.A | 0,37 |
| | C.B | 0,36 |
| | D.A | 0,37 |
| | D.B | 0,34 |

### Beispiel 2

Herstellung von Titandioxid-Dispersionen in Öl (Ansatzgröße 100 g):
Das Geminitensid und das Co-Tensid wurde im Öl Miglyol 812 (Hersteller Condea Chemie GmbH) bei 80 °C gelöst, bis eine homogene leicht trübe Dispersion entstand. Nach Abkühlen der Tensidphase auf ca. 30°C wurde unter Rühren das Pigment in die Ölphase eingearbeitet. Anschließend wurde die Dispersion mit einem Ultra-Turrax-Gerät 5 Minuten bei maximaler Umfangsgeschwindigkeit homogenisiert.

| **Inhaltsstoffe** | **Gew.-% (m/m)** |
|---|---|
| Geminitensid | 6,00 |
| Co-Amphiphil, Cetylalkohol | 1,00 |
| Miglyol® 812 | 58,00 |
| UV Titan M212 | 35,00 |

Nach 24 h bei Raumtemperatur wurde eine Messung der Partikelgrößenverteilung durchgeführt.

| **Pigment** | **Geminitensid** | **Median / Partikelgrößenverteilung (µm)** |
|---|---|---|
| Titanoxid UV Titan M212 | A.A | 0,45 |
| (Kemira) | A.B | 0,37 |
| | A.C | 0,44 |
| | A.D | 0,42 |
| | A.E | 0,38 |
| | B.A | 0,45 |
| | B.B | 0,37 |
| | C.A | 0,44 |
| | C.D | 0,43 |
| | D.A | 0,44 |
| | D.D | 0,43 |

### Beispiel 3

Herstellung von Zinkoxid-Dispersionen in Öl (Ansatzgröße 100 g):
Das Geminitensid und das Co-Amphiphil wurden im Öl Miglyol® 812 (Hersteller Condea Chemie GmbH) bei 80°C gelöst, bis eine homogene leicht trübe Dispersion entstand. Nach Abkühlen der Tensidphase auf ca. 30 °C wurde unter Rühren das Pigment in die Ölphase eingearbeitet. Anschließend wurde die Dispersion mit einem Ultra-Turrax-Gerät 5 Minuten bei maximaler Umfangsgeschwindigkeit homogenisiert.

| **Inhaltsstoffe** | **Gew% (m/m)** |
|---|---|
| Geminitensid | 4,50 |
| Co-Amphiphil, Cetylalkohol | 0,80 |
| Miglyol 812 | 44,70 |
| Z-Cote HP 1 | 50,00 |

Nach 24 h bei Raumtemperatur wurde eine Messung der Partikelgrößenverteilung durchgeführt.

| **Variante A Pigment** | **Geminitensid** | **Median / Partikelgrößenverteilung (µm)** |
|---|---|---|
| Zinkoxid Z-Cote HP 1 | A.A | 0,41 |
| (Hersteller Sun Smart) | A.B | 0,37 |
| | A.C | 0,41 |
| | A.D | 0,40 |
| | A.E | 0,45 |
| | B.A | 0,41 |
| | B.D | 0,40 |
| | C.A | 0,42 |
| | C.B | 0,38 |
| | D.A | 0,42 |
| | D.B | 0,38 |

### Beispiel 4

Als Komplexemulgator wurde eine Kombination aus Geminitensid, Glycerylsteara Glycerylstearatcitrat und Fettalkohol eingesetzt:

| **Komplexemulgator A.A** | **[Gew:-%]** | **Rezeptur** | **[Gew:-%]** |
|---|---|---|---|
| Geminitensid A.A | 10,00 | Komplexemulgator A.A | 1,90 |
| Glycerinmono-distearat | 35,00 | Miglyol 812 | 63,10 |
| Behenylalkohol | 40,00 | UV Titan M212 | 35,00 |
| Glycerylstearatcitrat | 15,00 | | |

### Herstellung:

- Emulgator im Öl bei 80°C lösen, auf 30°C abkühlen lassen
- Pigment langsam einarbeiten und für 15 min bei 1500 U/min dispergieren.

| Analysendaten (PSA = Partikelgrößenanalyse) | | |
|---|---|---|
| | **D-3-2/0** | **Tioveil Fin** |
| **PSA** | | |
| Median [µm] | 0,35 | 0,33 |
| < 1µm [%] | 96,5 | 92,0 |
| Median [µm] | 0,34 | 0,35 |
| <1µm [%] | 96,4 | 91,6 |

| **Rheologie** | | |
|---|---|---|
| Fließpunkt [mPa] | 5.000 | n.b. |
| Viskosität (bei 1 s⁻¹) [mPas] | 31.200 | n.b. |

### Beispiel 5 (erfindungsgemäße Verwendung)

Gemäß der nachfolgenden Rezeptur war es auch möglich, hydrophob gecoatetes Zinkoxid zu dispergieren.

| **Rezeptur** | **[Gew.-%]** |
|---|---|
| Komplexemulgator A.A | 2,30 |
| Miglyol 812 | 47,70 |
| Z-Cote HP 1 | 50,00 |

### Herstellung

- Emulgator im Öl bei 80°C lösen, auf 30°C abkühlen lassen
- Pigment langsam einarbeiten und für 15 min bei 1500 U/min dispergieren.

Auch bei diesem System wurde die Eignung des Komplexemulgators als effizientes Dispergiermittel in Öl unter Beweis gestellt:

| Analysendaten (PSA = Partikelgrößenanalyse) | |
|---|---|
| | **D-3-4/0** |
| **PSA** | |
| Median [µm] | 0,38 |
| < 1µm [%] | 84,0 |
| Median [µm] | 0,38 |
| <1µm [%] | 86,1 |

| **Rheologie** | |
|---|---|
| Fließpunkt [mPa] | 14.100 |
| Viskosität (bei 1s⁻¹) [mPas] | 39.200 |

### Emulsionen

### Beispiel 6 (Vergleichsbeispiel)

| Konventionelle Herstellung von O/W-Emulsionen: | | | |
|---|---|---|---|
| **Handelsname** | **Hersteller** | **CTFA/INCI- Nomenklatur** | **Gew.-%** |
| Phase A | | | |
| Tego Care 450 | Th. Goldschmidt | Polyglyceryl-3 methyl glucose distearat | 5,00 |
| ***Alternativ:** Geminitensid A.F oder B.C jeweils* | *Condea* | *Cetyl alcohol* | *4,00* |
| *plus Lanette 16* | *Henkel* | | *1,00* |
| Miglyol 812 N | Condea | Caprylic / capric triglyceride | 6,00 |
| Crodamol OP | Croda | 2-Ethylhexyl palmitate | 2,00 |
| Eutanol G | Henkel | Octyldodecanol | 2,00 |
| Softisan 100 | Condea | Hydrogenated coco-clycerides | 3,00 |

| Phase B | | | |
|---|---|---|---|
| Pricerina 9091 | Unichema | Glycerin | 3,00 |
| Demin. Wasser | | Aqua | 78,50 |

| Phase C | | | |
|---|---|---|---|
| Phenonip | Nipa | Phenoxyethanol Methylparaben, Propylparaben, Butylparaben | 0,50 |
| **Summe** | | | **100,00** |
| Gemini A.F: R¹ = R³= C₁₁H₂₃-/C₁₃H₂₇-1 R²= C₂H₄, X = Y = (C₂H₄O-)ₓ(C₃H₆O-)_{y} ; SO₃Na mit x = 17, y = 0 (A.I) | | | |

### Herstellung der Emulsion nach konventionellem Verfahren:

- Die Phasen A und B wurden getrennt auf 75 °C erwärmt,
- anschließend wurde die Phase A bei 75°C in die Phase B einemulgiert und 1 Minute bei 75 °C homogenisiert,
- danach wurde die Emulsion unter leichtem Rühren auf Raumtemperatur abgekühlt.

Die O/W-Emulsion mit Tego Care 450 wies eine Viskosität von 20.600 mPa s (Schergeschwindigkeit 1 s⁻¹, 25 °C) und eine durchschnittliche Tröpfchengröße zwischen 2 und 6 µm auf.

Die O/W-Emulsion hergestellt aus Gemini A.F und Cetylalkohol wies eine Viskosität von < 1000 mPa s (Schergeschwindigkeit 1 s⁻¹, 25 °C) und eine unbefriedigende Tröpfchengröße auf. Die O/W-Emulsion hergestellt aus Gemini B.C und Cetylalkohol erreichte lediglich eine Viskosität von < 1300 mPa s (Schergeschwindigkeit 1 s⁻¹, 25 °C).

### Beispiel 7

| Herstellung von O/W-Emulsionen nach der PTT-Methode | | | |
|---|---|---|---|
| **Handelsname** | **Hersteller** | **CTFA / INCI - Nomenklatur Gew%** | |
| Phase A *(mit Tego Care 450 nicht erfindungsgemäß)* | | | |
| Tego Care 450 | Th. Goldschmidt | Polyglyceryl-3 methyl glucose distearat | 5,00 |
| ***Alternativ:*** | | *(Alternative erfindungsgemäß)* | |
| *Gemini A. F oder B. C plus Lanette 16* | *Condea Henkel* | *Cetyl alcohol* | *4,00 1,00* |
| Miglyol 812 N | Condea | Caprylic / capric triglyceride | 10,00 |

| Phase B | | | |
|---|---|---|---|
| Pricerina 9091 | Unichema | Glycerin | 1,50 |
| Demin. Wasser | | Aquar | 18.50 |

| Phase C | | | |
|---|---|---|---|
| Pricerina 9091 | Unichema | Glycerin | 1,50 |
| Demin. Wasser | | | 60,00 |

| Phase D | | | |
|---|---|---|---|
| Phenonip | Nipa | Phenoxyethanol Methylparaben, Propylparaben, Butylparaben | 0,50 |
| **Summe** | | | **100,00** |
| Gemini A.F R¹ = R³= C₁₁H₂₃-/C₁₃H₂₇-1 R²= C₂H₄, X = Y = (C₂H₄O-)ₓ(C₃H₆O-)_{y} SO₃Na mit x = 17, y = 0 (A.I) | | | |

### Herstellung der Emulsion nach der PTT-Methode:

Die Phase A wurde auf 60 °C erwärmt, getrennt hiervon wurde die Phase B auf 50 °C erwärmt. Dann wurde die Phase A langsam unter starkem Homogenisieren in die Phase B einemulgiert und 1 Minute homogenisiert. Danach wurden unter langsamen Rühren die Phasen C und D homogen eingerührt.

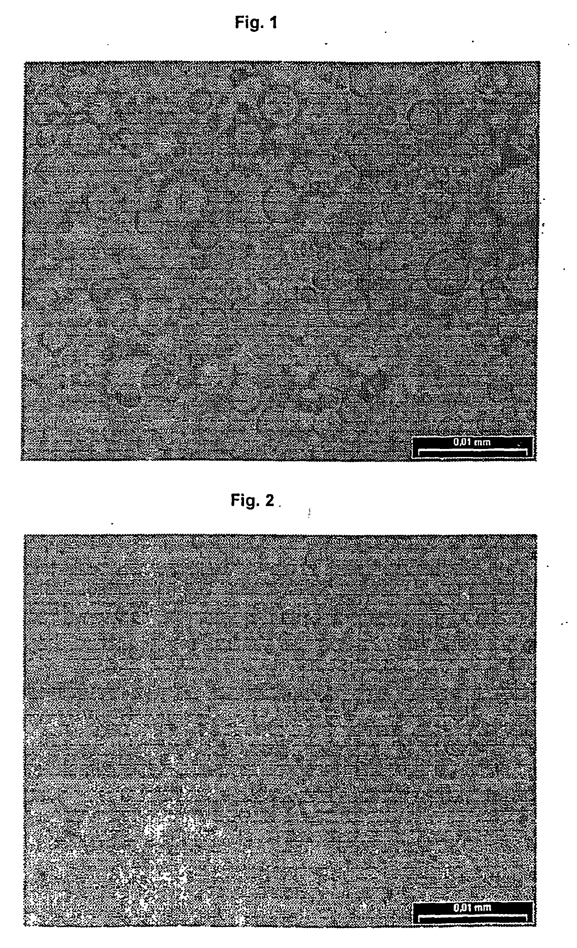

Die O/W-Emulsion mit Tego Care® 450 erreicht eine Viskosität von 20.000 mPas (Schergeschwindigkeit 1 s⁻¹, 25°C) und eine durchschnittliche Tröpfchengröße zwischen 2 und 6 µm. In der Fig. 1 ist die O/W-Emulsion mit Tego Care® 450 in 1100-facher Vergrößerung (Normarski Interferenz-Prisma, Viskosität 20.00 mPas, Schergeschwindigkeit 1 s⁻¹, 25°C) abgebildet. Die durchschnittliche Tröpfchengröße liegt zwischen 2 und 6 µm.

In der Fig. 2 ist die O/W-Emulsion mit Gemini A.F und Cetylalkohol bei 1100-facher Vergrößerung (Normarski Interferenz-Prisma, Viskosität 18.000 mPa s, Schergeschwindigkeit 1 s⁻¹, 25°C) abgebildet. Die O/W-Emulsion mit Gemini B.C und Cetylalkohol erreichte eine Viskosität von 18.000 mPa s (Schergeschwindigkeit 1 s⁻¹, 25°C) bei einer Tröpfchengröße von << 1 µm.

Anhand der Abbildungen ist offensichtlich, daß nach der PTT-Methode mit den erfindungsgemäßen Tensidzusammensetzungen Emulsionen herstellbar sind, die den konventionellen O/W-Emulsionen hinsichtlich der Feinteiligkeit deutlich überlegen und hinsichtlich der Rheologie mindestens ebenbürtig sind. Die Herstellung von Nanoemulsionen unter sehr milden Bedingungen mit apparativ geringem Aufwand ist also mit den erfindungsgemäßen Tensidzusammensetzungen möglich. Mit den erfindungsgemäßen Tensidzusammensetzungen hergestellte Emulsionen führen weiterhin zu Emulsionen mit einem seidigen Hautgefühl.

### Beispiel 8: Prüfung auf Hautirritationen beim Menschen im modifizierten Duhring-Kammer-Test

Nach der oben beschriebenen PTT-Methode wurden die folgenden Rezepturen hergestellt und nach der Methode von Frosch und Kligman dermatologisch bewertet (P.J. Frosch, A.M. Kligman, The Duhring Chamber, Contact Dermatitis, 5 (1979) 73-81; P.J. Frosch, A.M. Kligman, The soap chamber test, J. Am. Acad. Dermatol., 1 (1979) 35-41).

| Modellrezepturen für Duhring-Kammer-Test | | | | |
|---|---|---|---|---|
| **Rezeptur-Nr.** | **0-1** | **0-2** | **0-3** | **0-4** |
| **Handelsname** | | nicht erfindungsgemäß | nicht erfindungsgemäß | |
| **Phase A** | | | | |
| Tegin M | 3,00 | 3,00 | 3,00 | 3,00 |
| Gemini A.F | 1,00 | 0,00 | 0,00 | 0,00 |
| Gemini A.B | 0,00 | 0,00 | 0,00 | 1,00 |
| Emulgin B1 | 0,00 | 0,00 | 0,50 | 0,00 |
| Emulgin B2 | 0,00 | 0,00 | 0,50 | 0,00 |
| Emulgade PL1618 | 0,00 | 2,00 | 0,00 | 0,00 |
| Lanette O | 1,50 | 0,50 | 1,50 | 1,50 |

| **Phase B** | | | | |
|---|---|---|---|---|
| Cetiol V | 9,00 | 9,00 | 9,00 | 9,00 |
| Cetiol SN | 9,00 | 9,00 | 9,00 | 9,00 |

| **Phase C** | | | | |
|---|---|---|---|---|
| Wasser | 23,50 | 23,50 | 23,50 | 23,50 |
| Glycerin | 1,50 | 1,50 | 1,50 | 1,50 |

| **Phase D** | | | | |
|---|---|---|---|---|
| Wasser | 47,35 | 47,35 | 47,35 | 47,35 |
| Glycerin | 1,50 | 1,50 | 1,50 | 1,50 |

| **Phase D1** | | | | |
|---|---|---|---|---|
| Keltrol | 0,15 | 0,15 | 0,15 | 0,15 |

| **Phase E** | | | | |
|---|---|---|---|---|
| **Summe** | **100,00** | **100,00** | **100,00** | **100,00** |

0,05 ml des Produktes wurden auf markierte Stellen der Volarseite des Unterarms mit Hilfe von Aluminiumkammern (Finn Chambers, 12 mm Durchmesser), die entsprechende Filterpapiere enthielten, nach folgendem Zeitplan appliziert:

| | |
|---|---|
| 1. Tag: | 18 h Applikation - 6 h Pause |
| 2., 3., 4., 5. und 6. Tag | 6 h Applikation - 18 h Pause |
| 7. Tag: | Pause |
| 8. Tag: | Bewertung |

Als Kontrolle wurde als nicht-hautreizende Substanz Wasser und als irritierendes Mittel Natriumlaurylsulfat *(0,2* %) gewählt. Die Bewertung erfolgte mit Hilfe von drei verschiedenen Methoden, der visuellen Bewertung (in fünf Stufen), der Chromametrie und der Messung des transepidermalen Wasserverlustes als Marker für Barriereschäden. Die Beurteilung der Testsubstanzen erfolgte im Vergleich zur Kontrolle als nicht irritierend, schwach irritierend, mittelstark irritierend oder stark irritierend.

Alle vier Testprodukte erwiesen sich, sowohl was den Rötungsgrad als auch den transepidermalen Wasserverlust angeht, als "nicht irritierend" und wichen bei der geschilderten Auslegung des Testes statistisch nicht signifikant voneinander ab. Natriumlaurylsufat dagegen bewirkte die erwarteten irritativen Reaktionen, womit die Eignung des Testverfahrens und der korrekte Verlauf nachgewiesen war.

## Patentansprüche

1. Tensidzusammensetzung enthaltend
(A) zu 1 bis 70 Gew.%, vorzugsweise 10 bis 60 Gew.%, bezogen auf die Komponenten (A) und (B), ein oder mehrere Geminitenside und
(B) zum verbleibenden Rest, bezogen auf die Summe der Komponente (A) und (B), mehrere Co-Amphiphile, wobei die Co-Amphiphile einen HLB-Wert von kleiner oder gleich 6 aufweisen und ausgewählt sind aus zumindest 2 der unten genannten Co-Amphiphil-Komponenten (a) bis (d):
(a) einen oder mehrere längerkettige Alkohole:
- ein C6- bis C40- Alkohol,
(b) eine oder mehrere längerkettige Säuren:
- eine C6- bis C24-Carbonsäure,
(c) einen oder mehrere Ester/Teilester eines Polyols mit einer oder mehreren Mono- oder Poly-Carbonsäuren:
- ein Sorbitan(C6- bis C22-)ester,
- ein Methylglucosid(C6- bis C22-)ester,
- ein Zucker(C6- bis C22-)ester,
- ein Mono-, Di- und Triglycerid einer C6- bis C22- Carbonsäure,
- ein mit Milchsäure oder Zitronensäure verestertes Derivat der Mono- und Diglyceride einer C6- bis C22-Carbonsäure,
- ein Polyglycerin(C6- bis C22-)ester,
- ein Propylenglycol(C6- bis C22- )ester und/oder
- ein Vitaminester,
(d) sowie folgende weitere Co-Amphiphil-Komponenten:
- Salicylsäure,
- Benzoesäure und / oder
- Lecithin,
wobei zumindest ein Co-Amphiphil
- ein C6- bis C40- Alkohol und/oder
- ein Mono-, Di- und Triglycerid einer C6- bis C22- Carbonsäure ist.

2. Tensidzusammensetzung gemäß Anspruch 1, **dadurch gekennzeichnet, daß** die Tensidzusammensetzung
(A) zu 5 bis 60 Gew.%, vorzugsweise zu 10 bis 50 Gew.-%, bezogen auf die Komponenten (A) und (B), ein oder mehrere Geminitenside und
(B) zum verbleibenden Rest, bezogen auf die Summe der Komponente (A) und (B), mehrere Co-Amphiphile enthält.

3. Tensidzusammensetzung gemäß einem der vorhergehenden Ansprüche enthaltend weiterhin
(C) zu mindestens 0,1 Gew.%, bezogen auf die Gesamtzusammensetzung, Wasser.

4. Tensidzusammensetzung gemäß einem der vorhergehenden Ansprüche enthaltend weiterhin
(D) zu mindestens 0,1 Gew.%, bezogen auf die Gesamtzusammensetzung, eine oder mehrere Ölkomponenten.

5. Tensidzusammensetzung gemäß einem der vorhergehenden Ansprüche, wobei zumindest ein Co-Amphiphil
- eine C6- bis C24-, bevorzugt C8- bis C22-, Carbonsäure und/oder
- ein mit Milchsäure oder Zitronensäure verestertes Derivat der Mono- und Diglyceride einer C6- bis C22-Carbonsäure ist.

6. Tensidzusammensetzung gemäß einem der vorhergehenden Ansprüche in Form einer Emulsion, **dadurch gekennzeichnet, daß** das Co-Amphiphil bei 25°C im festen Zustand vorliegt.

7. Tensidzusammensetzung in Form einer Dispersion gemäß einem der Ansprüche 1 bis 5, **dadurch gekennzeichnet, daß** das Co-Amphiphil bei 25°C im flüssigen Zustand vorliegt.

8. Tensidzusammensetzung gemäß einem der vorhergehenden Ansprüche, **dadurch gekennzeichnet, daß** 3 bis 5 verschiedene Typen von Co-Amphiphilen eingesetzt werden.

9. Tensidzusammensetzung gemäß einem der vorhergehenden Ansprüche, wobei zumindest ein Co-Amphiphil
- ein C6- bis C40-, bevorzugt C8- bis C24-, Alkohol ist.

10. Tensidzusammensetzung gemäß einem der vorhergehenden Ansprüche, **dadurch gekennzeichnet, daß** die Tensidzusammensetzung
- 30 bis 50 Gew.% eines C6- bis C40-, bevorzugt C8- bis C24-, Alkohols und/oder
- 30 bis 50 Gew.% eines Mono-, Di- und Triglycerids einer C6- bis C22- Carbonsäure,
jeweils bezogen auf die Zusammensetzung Geminitensid / Co-Amphiphil(e), enthält.

11. Tensidzusammensetzung nach einem der vorhergehenden Ansprüche in Form einer Emulsion, **dadurch gekennzeichnet, daß** die Tensidzusammensetzung herstellbar ist durch ein Verfahren (Phasentransfer-Temperatur (PTT)-Methode), das zumindest den folgenden Schritt aufweist:
Zusammenbringen
(a) einer Zusammensetzung (a) enthaltend oder bestehend aus dem Geminitensid (A), sowie ggf. Wasser, wobei die Zusammensetzung eine Temperatur X aufweist, mit
(b) einer Zusammensetzung (b) enthaltend oder bestehend aus dem Co-Amphiphil (B) sowie ggf. einer Ölkomponente, wobei die Zusammensetzung eine Temperatur Y aufweist,
wobei die Temperatur Y größer als die Temperatur X ist.

12. Tensidzusammensetzung nach Anspruch 11, **dadurch gekennzeichnet, daß** die Temperatur Y höchstens 15°C größer ist als die kritische Phasentransfertemperatur des Tensids in der Zusammensetzung (b) ist.

13. Tensidzusammensetzungen nach einem der Ansprüche 11 oder 12, **dadurch gekennzeichnet, daß** die Temperaturen X und Y um mindestens 3°C, vorzugsweise um mindestens 5°C, verschieden sind.

14. Tensidzusammensetzung nach einem der vorhergehenden Ansprüche, **dadurch gekennzeichnet, daß** das Geminitensid an der Verknüpfungsstelle zwischen Spacer, hydrophiler und hydrophober Gruppe Stickstoffatome aufweist.

15. Tensidzusammensetzung nach einem der vorhergehenden Ansprüche, **dadurch gekennzeichnet, daß** das Geminitensid einen Amin- oder Amidgruppen aufweisenden Spacer mit 1 bis 12 C-Atomen aufweist.

16. Tensidzusammensetzung nach einem der vorhergehenden Ansprüche, **dadurch gekennzeichnet, daß** die hydrophobe Doppelgruppe je einen C6- bis C24-Kohlenwasserstoffrest und/oder die hydrophile Doppel(Kopf-)gruppe einen mindestens einfach alkoxylierten Rest aufweist, der eine Sulfonsäure-, Carbonsäure-, Phosphonsäure-, Polyalkohol- oder Polyalkylenoxid - Gruppe trägt, die gegebenenfalls auch in Salzform vorliegen kann.

17. Tensidzusammensetzung nach einem der vorhergehenden Ansprüche, **dadurch gekennzeichnet, daß** die Tensidzusammensetzung 0,01 bis 30 Gew.%, vorzugsweise 0,1 bis 6 Gew.% der Komponenten (A) und (B) bezogen auf die Gesamtzusammensetzung enthält.

18. Tensidzusammensetzung nach einem der Ansprüche 1 bis 17, **dadurch gekennzeichnet, daß** das Geminitensid die allgemeine Formel (A.I) aufweist 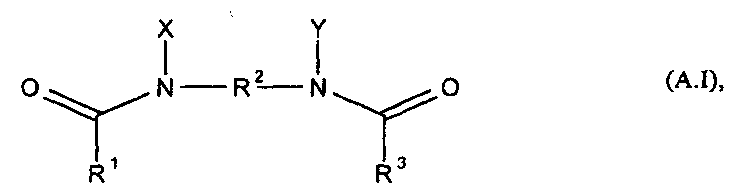 wobei die Substituenten folgende Bedeutung haben:
R¹, R³ C₅- bis C₂₅-Alkyl, verzweigt oder unverzweigt, gesättigt, gegebenenfalls bis zu 2-fach nicht-benachbart ungesättigt;
R² C₁- bis C₁₂-Alkylen;
X,Y (C₂H₄O-)ₓ(C₃H₆O-)_{y}-FR; x+y ≥ 1, x: 0-15, y: 0-10 und
FR -SO₃M, -CH₂-CO₂M, -P(O)(OM)₂, H, -C₃H₆SO₃M, -CH₂(CHOH)₄CH₂OH soweit x+y=0 , wobei M = Alkali, (Alkyl)Ammonium, Alkanolammonium, H oder ½ Erdalkali.

19. Tensidzusammensetzung nach einem der Ansprüche 1 bis 17, **dadurch gekennzeichnet, daß** das Geminitensid die allgemeine Formel (A.II) aufweist 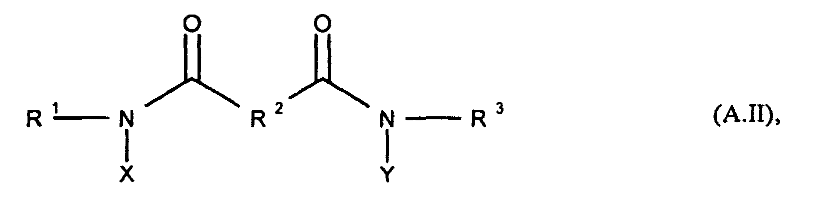 wobei die Substituenten die für die allgemeine Formel (A.I) in Anspruch 18 angegebene Bedeutung haben.

20. Tensidzusammensetzung nach einem der Ansprüche 1 bis 17, **dadurch gekennzeichnet, daß** das Geminitensid die allgemeine Formel (A.III) aufweist 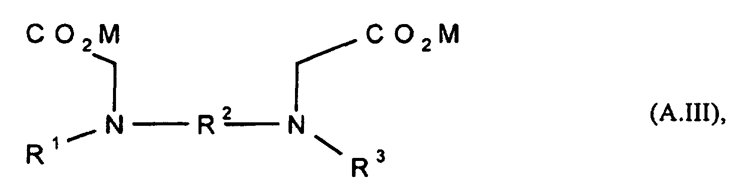 wobei die Substituenten die für die allgemeine Formel (A.I) in Anspruch 18 angegebene Bedeutung haben.

21. Tensidzusammensetzung nach einem der Ansprüche 1 bis 17 , **dadurch gekennzeichnet, daß** das Geminitensid die allgemeine Formel (B.I) aufweist 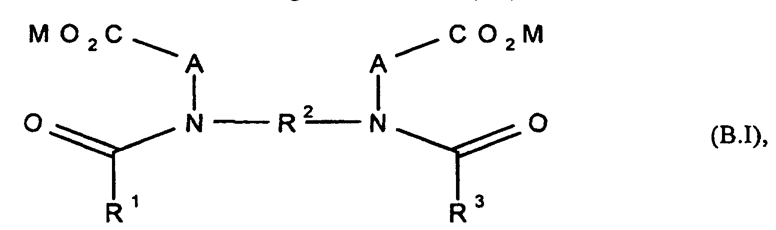 wobei die Substituenten folgende Bedeutung haben:
R¹, R³ C₅- bis C₂₅-Alkyl, verzweigt oder unverzweigt, gesättigt, gegebenenfalls bis zu 2-fach nicht-benachbart ungesättigt;
R² C₁- bis C₁₂-Alkylen;
A CHR⁴, CH₂, C₂H₄, C₃H₆, C₄H₈;
R⁴ Rest einer Aminocarbonsäure und
M Alkali, (Alkyl)Ammonium, Alkanolammonium, H oder ½ Erdalkali.

22. Tensidzusammensetzung nach einem der Ansprüche 1 bis 17, **dadurch gekennzeichnet, daß** das Geminitensid die allgemeine Formel (B.II) aufweist 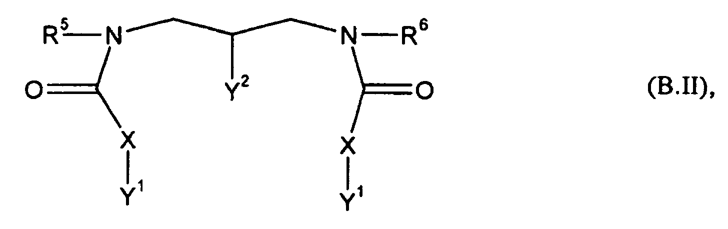 wobei
R⁵, R⁶ C₆- bis C₃₆-Alkyl, verzweigt oder unverzweigt, gesättigt, gegebenenfalls bis zu 2-fach nicht-benachbart ungesättigt;
X Alkylen- oder Alkenylengruppe mit 1 bis 6 Kohlenstoffatomen, die ggf. mit einer Hydroxylgruppe oder einer Sulfonsäuregruppe oder einer Carboxygruppe substituiert ist;
Y¹ eine Sulfonat- oder Sulfatgruppe oder eine Carboxylgrupppe und
Y² eine Hydroxylgruppe, ein Schwefelsäurerest oder -O-(CO)X-COOH bedeuten.

23. Tensidzusammensetzung nach einem der Ansprüche 1 bis 17, **dadurch gekennzeichnet, daß** das Geminitensid die allgemeine Formel (B.III) aufweist 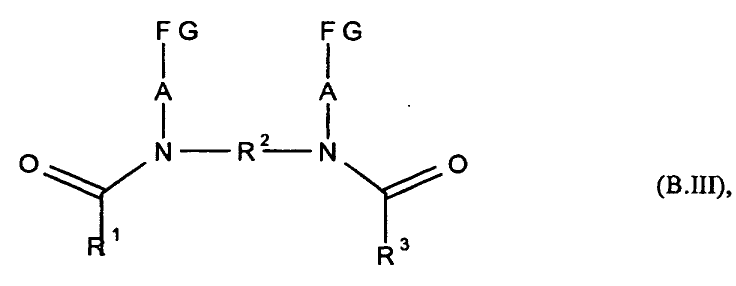 wobei die Substituenten die für die allgemeine Formel (B.I) in Anspruch 21 angegebene Bedeutung haben und
FG -COOM oder -SO₃M bedeutet.

24. Tensidzusammensetzung nach einem der Ansprüche 1 bis 17, **dadurch gekennzeichnet, daß** das Geminitensid die allgemeine Formel (B.IV) aufweist 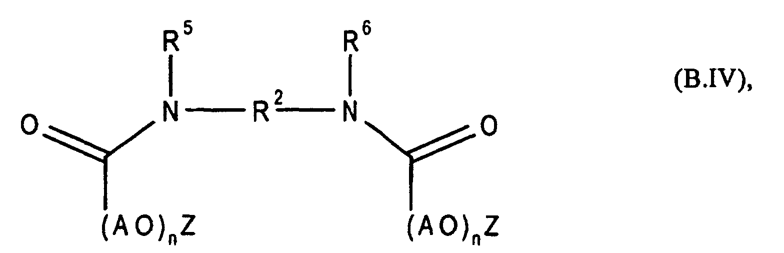 wobei die Substituenten, die bei den allgemeinen Formeln (B.I) und (B.II) gemäß den Ansprüchen 21 und 22 angegebene Bedeutung haben und
AO Alkylenoxideinheiten, d.h. Ethylenglykol-, Propylenglykol und Butylenglykolethereinheiten, allein oder statistisch oder blockweise verteilt, mit n = 1 bis 20 und
Z -SO₃M, -C₂H₄SO₃M, -C₃H₆SO₃M, -P(O)(OM)₂, -CH₂-COOM oder -C₂H₄-COOM bedeuten.

25. Tensidzusammensetzung nach einem der Ansprüche 1 bis 17, **dadurch gekennzeichnet, daß** das Geminitensid die allgemeine Formel (C.I) aufweist 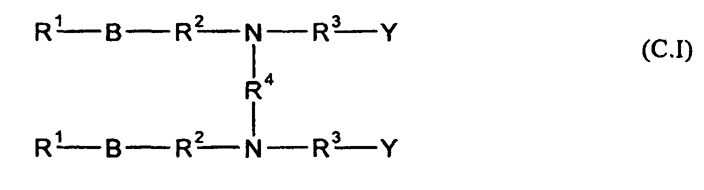 wobei die Substituenten folgende Bedeutung haben:
R¹ C₅- bis C₂₅-Alkyl, verzweigt oder unverzweigt, gesättigt, gegebenenfalls bis zu 2-fach nicht-benachbart ungesättigt, hydroxysubstituiert oder perfluoriert ;
R² C₁- bis C₁₂-Alkylen oder hydroxysubstituierte Derivate davon;
B eine Amidgruppe [-C(O)N(R²)- oder -N(R⁵)C(O)-], eine Carboxylgruppe [-C(O)O- oder -OC(O)-], eine Polyethergruppe [-(O(R⁶-O)x-];
R⁵ für C₁- bis C₄-Alkyl oder hydroxysubstituiertes Alkyl oder H steht;
R⁶ für C₂- bis C₄-Alkylen;
x eine Zahl von 1 bis 20;
R³ für C₁- bis C₁₂- Alkyl oder hydroxysubstituierte Derivate davon, R⁷-D-R⁷ oder eine Polyethergruppe [-(O(R⁶-O)x-];
R⁷ für C₁- bis C₆- Alkylen oder hydroxysubstituierte Derivate davon;
D -O-, -S-, -N(R⁸)- ;
R⁴ Alkylen oder Alkylaryl mit 1 bis 12 C-Atomen oder die hydroxysubstituierten Derivaten oder R⁹-D¹-R⁹ ;
R⁸ C₁- bis C₁₂-Alkyl oder hydroxysubstituiertes Alkyl oder H oder R⁹-D¹-R⁹;
R⁹ für C₁- bis C₆- Alkylen oder hydroxysubstituierte Derivate davon oder Aryl;
D¹ -O-, -S-, -SO₂-, -C(O)-, [-(O(R⁷-O)x-], (R¹⁰)t[N(R¹⁰)]z oder Aryl;
R¹⁰ C₁- bis C₁₂-Alkyl oder hydroxysubstituiertes Alkyl oder H oder Aryl;
t,z unabhängig voneinander eine Zahl von 1 bis 4 bedeuten und
Y unabhängig voneinander für -SO₃H, O-SO₃H, -OP(O)(OH)₂, -P(O)(OH)₂, -COOH, -CO₂-C₆H₄-SO₃H oder deren Salze steht.

26. Tensidzusammensetzung nach einem der Ansprüche 1 bis 17, **dadurch gekennzeichnet, daß** das Geminitensid die allgemeine Formel (C.II) aufweist 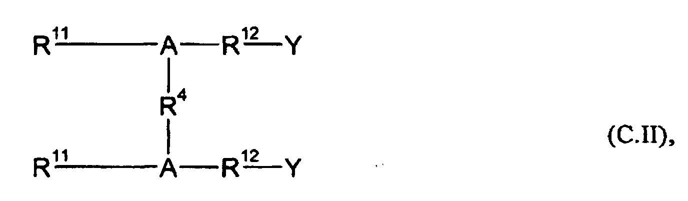 wobei die Substituenten die für die allgemeine Formel (C.I) in Anspruch 25 angegebene Bedeutung haben und
R¹¹ C₅- bis C₂₃-Alkyl, verzweigt oder unverzweigt, gesättigt, gegebenenfalls bis zu 2-fach nicht-benachbart ungesättigt, hydroxysubstituiert oder perfluoriert oder R¹⁴-B-R²;
R¹⁴ C₁- bis C₁₂-Alkyl, verzweigt oder unverzweigt, gesättigt, gegebenenfalls bis zu 2-fach nicht-benachbart ungesättigt, oder die hydroxysubstituierten Derivate;
R¹² C¹- bis C¹²-Alkylen, verzweigt oder unverzweigt, gesättigt, gegebenenfalls bis zu 2-fach nicht-benachbart ungesättigt, oder die hydroxysubstituierten Derivate oder eine Amidgruppe [-C(O)N(R²)- oder -N(R⁵)C(O)-], eine Carboxylgruppe [-C(O)O- oder -OC(O)-], eine Polyethergruppe [-(O(R⁶-O)x-] oder R⁹-D¹-R⁹ und
A -CR⁶= oder -N= unter der Voraussetzung, daß wenn A gleich -N= ist, R¹¹ gleich R¹⁴-B-R² bedeuten.

27. Tensidzusammensetzung nach einem der Ansprüche 1 bis 17, **dadurch gekennzeichnet, daß** das Geminitensid die allgemeine Formel (C.III) aufweist 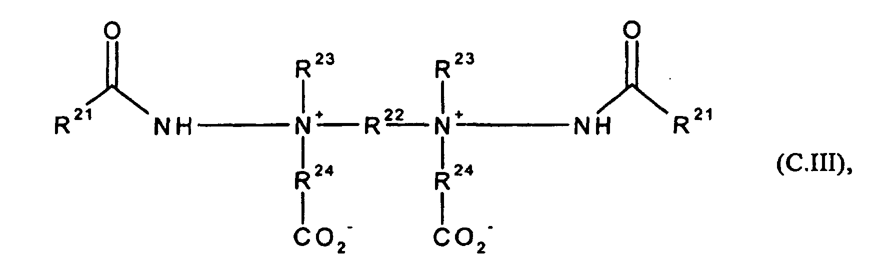 wobei
R²¹ C₅- bis C₂₃-Alkyl, verzweigt oder unverzweigt, gesättigt, gegebenenfalls bis zu 2-fach nicht-benachbart ungesättigt;
R²², R²⁴ C₁- bis C₆ Alkylen und
R²³ Methyl, Ethyl, Propyl oder eine Polyethergruppe [-(O(R⁶-O)ₓ-] bedeuten.

28. Tensidzusammensetzung nach einem der Ansprüche 1 bis 13, 16 oder 17, **dadurch gekennzeichnet, daß** das Geminitensid die allgemeine Formel (D.I) aufweist 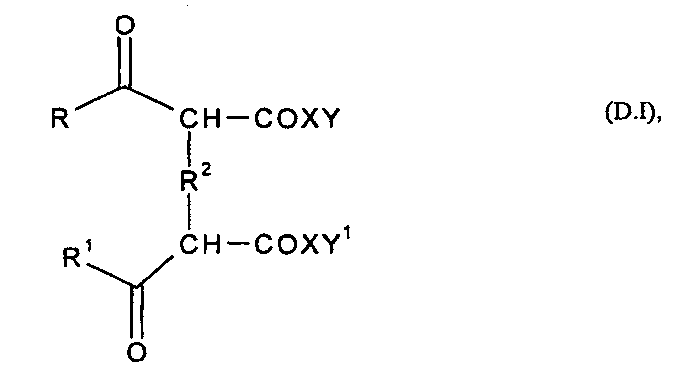 wobei die Substituenten folgende Bedeutung haben:
R, R¹ C₅- bis C₃₀-Alkyl, verzweigt oder unverzweigt, gesättigt, gegebenenfalls bis zu 2-fach nicht-benachbart ungesättigt, hydroxysubstituiert oder perfluoriert;
R² C₁-bis C₁₀- Alkylen, Arylen odere deren hydroxysubstituierte Derivate, ein Polyether [-O(R⁴O)ₓ-], -S-, -SO₂-, -O-, -S-S-, -O-R⁵-O- oder -S-R⁵-S-; Variable für eine direkte Bindung zwischen den beiden α-Kohlenstoffen;
R⁴ C₂- bis C₄-Alkylen;
R⁵ C₁- bis C₁₀-Alkylen, Arylen oder Alkylarylen, -N(R⁶)- oder -(NR⁶)-R⁷-(NR⁶) ;
R⁶ C₁- bis C₆-Alkyl;
R⁷ C₁- bis C₆-Alkyl, wobei R⁷ und R⁶ auch Teil eines heterocyclischen Ringes sein können;
X Polyether [-O(R⁴O)ₓ-], wobei x eine Zahl von 1 bis 30 ist, -O-, NZ;
Z C₁- bis C₁₀- Alkyl, Aryl, Alkylaryl oder H und
Y, Y¹ unabhängig voneinander H, -CH2-COOH und Salze, Kohlenwasserstoffrest mit mindestens 2 Hydroxylgruppen, wie Erythrose, Threose, Ribose, Arabinose, Xylose, Fructose, Lyxose, Allose, Altrose, Glucose, Mannose, Galactose und ihre Mischungen.

29. Tensidzusammensetzung nach einem der Ansprüche 1 bis 13, 16 oder 17, **dadurch gekennzeichnet, daß** das Geminitensid die allgemeine Formel (D.II) aufweist 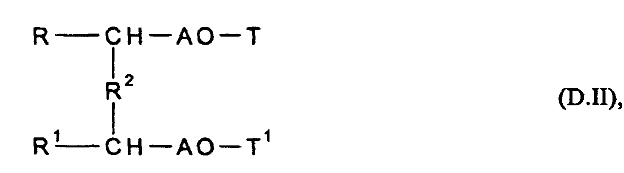 wobei die Substituenten die für die allgemeine Formel (D.I) in Anspruch 28 angegebene Bedeutung haben und
AO -C(O)-, -C(O)- [-O(R⁴O)ₓ-], -CH₂- [-O(R⁴O)ₓ-], -CH₂-O-;
T, T1 unabhängig voneinander -OM, -H, -CH₃, -C₂H₅, -SO₃M, -CH₂COOM, -C₂H₄-COOM, -C₃H₆-SO₃M, -O-P(O)(OM)₂ und
M Alkyli, ½ Erdalkali, Ammonium, Mono-, Di-, Trialkanolammonium oder H bedeuten.

30. Tensidzusammensetzung nach einem der Ansprüche 1 bis 17, **dadurch gekennzeichnet, daß** das Geminitensid die allgemeine Formel (D.III) aufweist 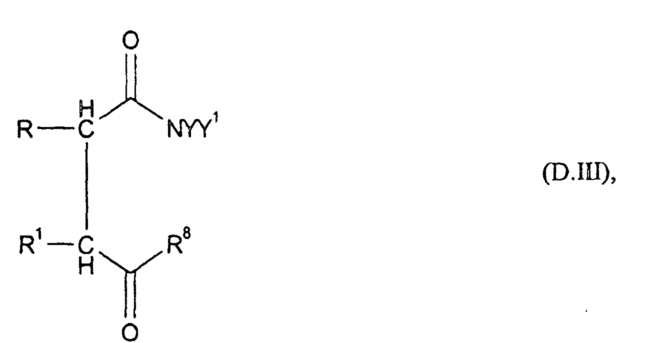 wobei die Substituenten die für die allgemeinen Formeln (D.I) und (D.II) gemäß den Ansprüchen 28 und 29 angegebene Bedeutung haben und
R⁸ NYY¹, -O(R⁴O)ₓH oder -O(R⁴O)ₓ-C(O)-CHR-CHR¹-C(O)NYY¹ bedeutet.

31. Tensidzusammensetzung nach einem der Ansprüche 1 bis 13, 16 oder 17, **dadurch gekennzeichnet, daß** das Geminitensid die allgemeine Formel (D.IV) aufweist 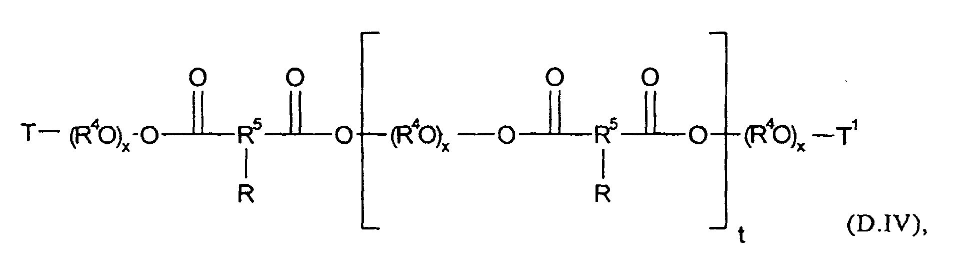 wobei die Substituenten die für die allgemeinen Formeln (D.I), (D.II) und (D.III) gemäß den Ansprüchen 28-30 angegebene Bedeutung haben und
t eine ganze Zahl von 1 bis 100, bevorzugt 1 bis 20, besonders bevorzugt 1 bis 4 bedeutet.

32. Verwendung der Tensidzusammensetzung nach einem der vorhergehenden Ansprüche als Emulgier- oder Dispergiermittel, als Zusatz zu konventionellen anionischen Tensiden oder als Zusatz zu Haar- und Hautreinigungsmittel-Formulierungen.

## Claims

1. A surfactant composition comprising
(A) 1 to 70 wt%, preferably 10 to 60 wt%, referring to components (A) and (B), of one or more gemini surfactant(s) and,
(B) referring to the remainder, based on the total of components (A) and (B), one or more co-amphiphile(s) having an HLB value of less than or equal to 6 and selected from at least two of the co-amphiphiles (a) to (d) as defined below:
(a) one or more long-chain alcohol(s):
- a C₆- to C₄₀- alcohol,
(b) one or more long-chain acid(s):
- a C₆- to C₂₄- carboxylic acid,
(c) one or more ester(s)/partial ester(s) of a polyol with one or more mono- or polycarboxylic acid(s):
- a sorbitan(C₆- to C₂₂-)ester,
- a methylglucoside(C₆- to C₂₂-)ester,
- a sugar(C₆- to C₂₂-)ester,
- a mono-, di- and triglyceride of a C₆- to C₂₂- carboxylic acid,
- a lactic acid or citric acid-esterified derivative of the mono- and diglycerides of a C₆- to C₂₂-carboxylic acid,
- a polyglycerol (C₆- to C₂₂-)ester,
- a propyleneglycol (C₆- to C₂₂-)ester,
- vitamin ester,
(d) and the following additional co-amphiphiles:
- salicylic acid,
- benzoic acid and/or
- lecithin,
wherein at least one co-amphiphile is:
- a C₆- to C₄₀- alcohol and/or
- a mono-, di- and triglyceride of a C₆- to C₂₂- carboxylic acid.

2. The surfactant composition of Claim 1, **characterized in that** the surfactant composition comprises
(A) 5 to 60 wt%, preferably 10 to 50 wt%, referring to components (A) and (B), of one or more gemini surfactant(s) and,
(B) referring to the remainder, based on the total of components (A) and (B), of more co-amphiphile(s).

3. A surfactant composition according to any one of the preceding claims further comprising
(C) at least 0.1 wt% water, referring to the total composition.

4. A surfactant composition according to any one of the preceding claims further comprising
(D) at least 0.1 wt% of one or more oil component(s), referring to the total composition.

5. A surfactant composition according to any one of the preceding claims, wherein at least one co-amphiphile is
- a C₆- to C₂₄-, preferably C₈- to C₂₂-carboxylic acid and/or
- a with lactic acid- or citric acid-esterified derivative of the mono- and diglycerides of a C₆- to C₂₂-carboxylic acid.

6. A surfactant composition according to any one of the preceding claims in form of an emulsion, **characterized in that** the co-amphiphile is present in solid form at 25°C.

7. A surfactant composition in form of a dispersion according to any one of claims 1 to 5, **characterized in that** the co-amphiphile is present in liquid form at 25°C.

8. A surfactant composition according to any one of the preceding claims, **characterized in that** 3 to 5 different co-amphiphiles are employed.

9. A surfactant composition according to any one of the preceding claims, wherein at least one co-amphiphile is
- a C₆- to C₄₀-, preferably C₈- to C₂₄-alcohol.

10. A surfactant composition according to any one of the preceding claims, **characterized in that** the surfactant composition comprises
- 30 to 50 wt% of C₆- to C₄₀-, preferably C₈- to C₂₄- alcohol, and/or
- 30 to 50 wt% of a mono-, di-, and triglyceride of a C₆- to C₂₂-carboxylic acid, each referring to the gemini surfactant/co-amphiphile(s) composition.

11. A surfactant composition according to any one of the preceding claims in form of an emulsion, **characterized in that** the surfactant composition can be produced by a method (phase transfer temperature (PTT) method), which includes at least the following step:
combining
(a) a composition (a) comprising of the gemini surfactant (A) and, optionally water, wherein the composition has a temperature X, with
(b) a composition (b) comprising the co-amphiphile (B) and, optionally an oil component, wherein the composition has a temperature Y,
the temperature Y being greater than temperature X.

12. The surfactant composition of claim 11, **characterized in that** the temperature Y is not more than 15°C higher than the critical phase transfer temperature of the surfactant in composition (b).

13. Surfactant compositions according to any one of claims 11 or 12, **characterized in that** the temperatures X and Y are different by at least 3°C, preferably at least 5°C.

14. A surfactant composition according to any one of the preceding claims, **characterized in that** the gemini surfactant comprises nitrogen atoms at the link between spacer, hydrophilic, and hydrophobic group.

15. A surfactant composition according to any one of the preceding claims, **characterized in that** the gemini surfactant comprises an amine- or amide-group-containing spacer with 1 to 12 carbon atoms.

16. A surfactant composition according to any one of the preceding claims, **characterized in that** the hydrophobic double group comprises a C₆- to C₂₄-hydrocarbon residue each and/or the hydrophilic double (head) group comprises an at least monoalkoxylated residue with a sulfonic acid-, carboxylic acid-, phosphonic acid-, polyalcohol-, or polyalkylene oxide group, which may also be present in salt form.

17. A surfactant composition according to any one of the preceding claims, **characterized in that** the surfactant composition comprises 0.01 to 30 wt%, preferably 0.1 to 6 wt% of the components (A) and (B), referring to the total composition.

18. A surfactant composition according to any one of claims 1 to 17, **characterized in that** the gemini surfactant has the general formula (A.I) 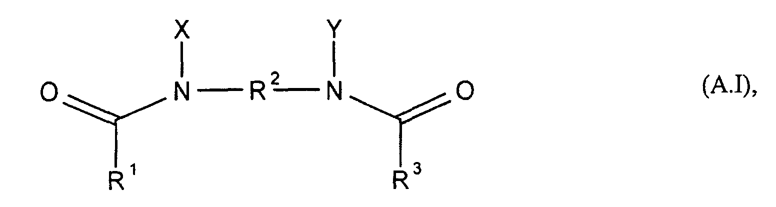 wherein the substituents have the following meanings:
**R**¹, **R**³ C₅- to C₂₅-alkyl, branched or unbranched, saturated, optionally as far as non-adjacently diunsaturated;
**R**² C₁- to C₁₂-alkylene;
**X, Y** (C₂H₄O-)ₓ(C₃H₆O-)_{y}-FR; x+y ≥ 1, x: 0-15, y: 0-10 ; and
**FR** -SO₃M, -CH₂-CO₂M, -P(O)(OM)₂, H, -C₃H₆SO₃M, -CH₂(CHOH)₄CH₂OH, insofar as x+y=0,
wherein M = alkali, (alkyl)ammonium, alkanol ammonium, H, or ½ alkaline earth.

19. A surfactant composition according to any one of claims 1 to 17, **characterized in that** the gemini surfactant has the general formula (A.II) 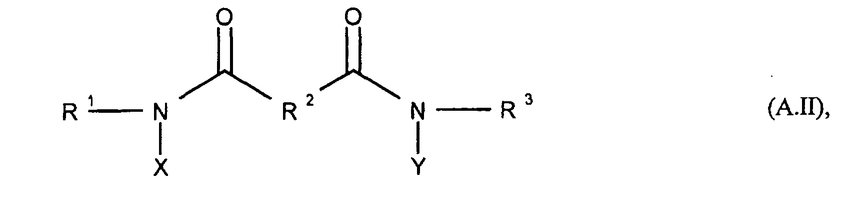 wherein the substituents have the meanings as defined by the general formula (A.I) of claim 18.

20. A surfactant composition according to any one of claims 1 to 17, **characterized in that** the gemini surfactant has the general formula (A.III) 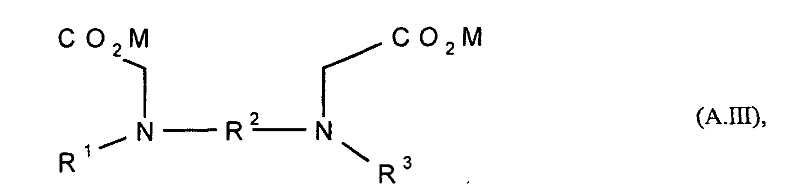 wherein the substituents have the meanings as defined by the general formula (A.I) of claim 18.

21. A surfactant composition according to any one of claims 1 to 17, **characterized in that** the gemini surfactant has the general formula (B.I) 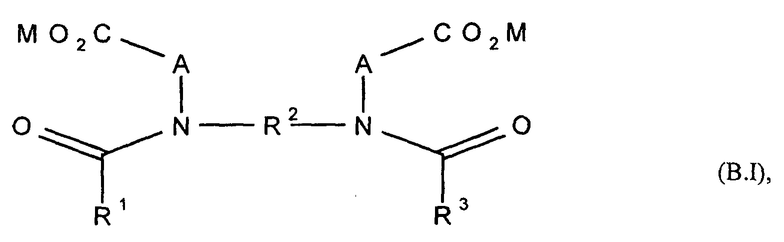 wherein the substituents have the following meanings:
**R**¹, **R**³ C₅- to C₂₅-alkyl, branched or unbranched, saturated, optionally as far as non-adjacently diunsaturated;
**R**² C₁- to C₁₂-alkylene
**A** CHR⁴, CH₂, C₂H₄, C₃H₆, C₄H₈;
**R**⁴ aminocarboxylic acid radical, and
**M** alkali, (alkyl)ammonium, alkanol ammonium, H, or ½ alkaline earth.

22. A surfactant composition according to any one of claims 1 to 17, **characterized in that** the gemini surfactant has the general formula (B.II) 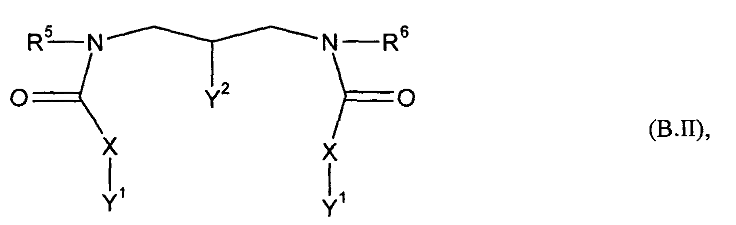 wherein
**R**⁵, R⁶ represent C₆- to C₃₆-alkyl, branched or unbranched, saturated, optionally as far as non-adjacently diunsaturated;
**X** is an alkylene- or alkenylene group having from 1 to 6 carbon atoms, which may be substituted with a hydroxyl, sulfonic acid or carboxy group;
**Y**¹ is a sulfonate- or sulfate group or a carboxyl group, and
**Y**² represents a hydroxyl group, a sulfuric acid residue, or -O-(CO)X-COOH.

23. A surfactant composition according to any one of claims 1 to 17, **characterized in that** the gemini surfactant has the general formula (B.III) 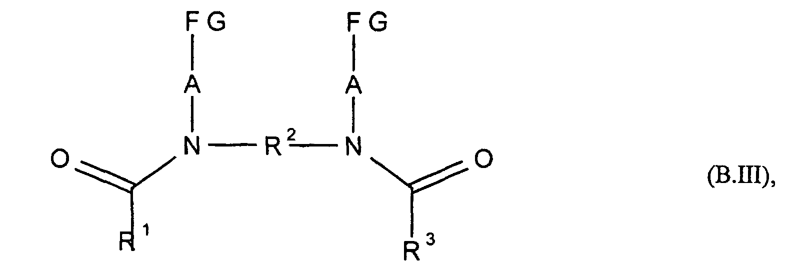 wherein the substituents have the meanings as defined by the general formula (B.I) of claim 21 and
**FG** represents -COOM or -SO₃M.

24. A surfactant composition according to any one of claims 1 to 17, **characterized in that** the gemini surfactant has the general formula (B.IV) 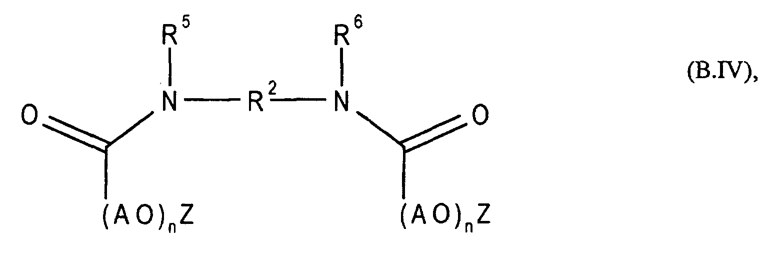 wherein the substituents have the meanings as defined by the general formulas (B.I) and (B.II) according to claims 21 and 22 and
**AO** represents alkylene oxide units, i.e. ethyleneglycol-, propyleneglycol-, and butyleneglycol ether units, alone or arranged randomly or blockwise, wherein n = 1 to 20, and
**Z** is -SO₃M, -C₂H₄SO₃M, -C₃H₆SO₃M, -P(O)(OM)₂, -CH₂-COOM, or -C₂H₄-COOM.

25. A surfactant composition according to any one of claims 1 to 17, **characterized in that** the gemini surfactant has the general formula (C.I), 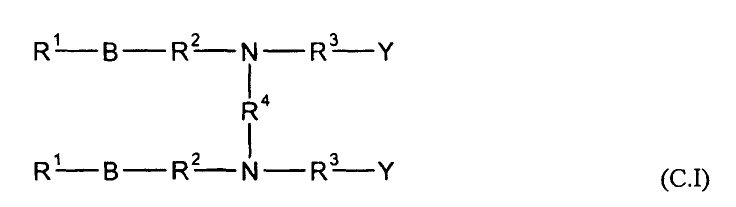 wherein the substituents have the following meanings:
**R**¹ C₅- to C₂₅-alkyl, branched or unbranched, saturated, optionally as far as non-adjacently diunsaturated, hydroxy-substituted or perfluorinated;
**R**² C₁- to C₁₂-alkylene or hydroxy-substituted derivatives thereof;
**B** an amide group [-C(O)N(R²)- or -N(R⁵)C(O)-], a carboxyl group [-C(O)O- or -OC(O)-], a polyether group [-O(R⁶-O)ₓ-];
**R**⁵ C₁- to C₄-alkyl or hydroxy-substituted alkyl or H;
**R**⁶ C₂- to C₄-alkylene;
**x** a number from 1 to 20;
**R**³ C₁- to C₁₂-alkyl or hydroxy-substituted derivatives thereof, R⁷-D-R⁷ or a polyether group [-O(R⁶-O)ₓ-];
**R**⁷ C₁- to C₆- alkylene or hydroxy-substituted derivatives thereof;
**D** -O-, -S-, -N(R⁸)-;
**R**⁴ alkylene or alkylaryl having from 1 to 12 carbon atoms or the hydroxysubstituted derivatives or R⁹-D¹-R⁹ ;
**R**⁸ C₁- to C₁₂-alkyl or hydroxy-substituted alkyl or H or R⁹-D¹-R⁹;
**R**⁹ C₁- to C₆-alkylene or hydroxy-substituted derivatives thereof or aryl;
**D**¹ -O-, -S-, -SO₂-, -C(O)-, [-O(R⁷-O)ₓ-], (R¹⁰)ₜ[N(R¹⁰)]_{z} or aryl;
**R**¹⁰ C₁- to C₁₂-alkyl or hydroxy-substituted alkyl or H or aryl;
**t, z** are independently a number from I to 4, and
**Y** is independently -SO₃H, -O-SO₃H, -OP(O)(OH)₂, -P(O)(OH)₂, -COOH, -CO₂-C₆H₄-SO₃H or the salts thereof.

26. A surfactant composition according to any one of claims 1 to 17, **characterized in that** the gemini surfactant has the general formula (C.II) 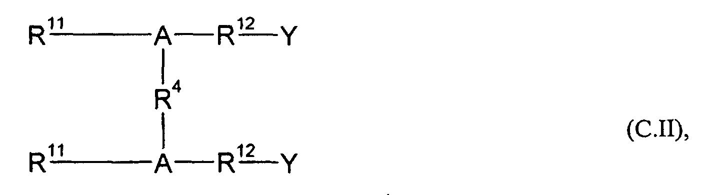 wherein the substituents have the meanings as defined by the general formula (C.I) of claim 25 and
**R**¹¹ is C₅- to C₂₃-alkyl, branched or unbranched, saturated, optionally as far as non-adjacently diunsaturated, hydroxy-substituted or perfluorinated or R¹⁴-B-R²;
**R**¹⁴ is C₁- to C₁₂-alkyl, branched or unbranched, saturated, optionally as far as non-adjacently diunsaturated, or the hydroxy-substituted derivatives;
**R**¹² means C₁- to C₁₂-alkylene, branched or unbranched, saturated, optionally as far as non-adjacently diunsaturated, or the hydroxy-substituted derivatives, or an amide group [-C(O)N(R²)- or -N(R⁵)C(O)-], a carboxyl group [-C(O)O- or -OC(O)-], a polyether group [-O(R⁶-O)ₓ-] or R⁹-D¹-R⁹ and
**A** is -CR⁶= or -N= , if whenever A is equal to -N= , R¹¹ represents R¹⁴-B-R².

27. A surfactant composition according to any one of claims 1 to 17, **characterized in that** the gemini surfactant has the general formula (C.III) 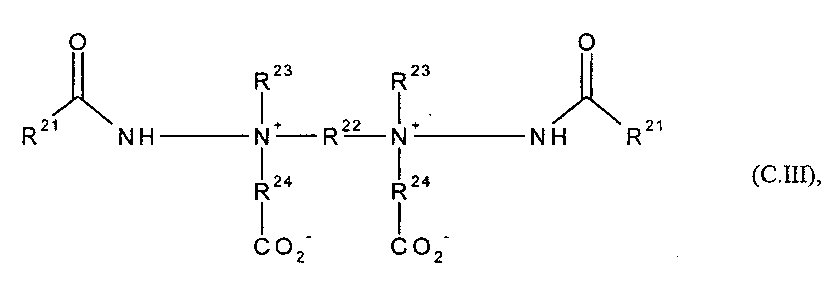 wherein
**R**²¹ represents C₅- to C₂₃-alkyl, branched or unbranched, saturated, optionally as far as non-adjacently diunsaturated;
**R**²², **R**²⁴ are C₁- to C₆-alkylene;
**R**²³ is methyl, ethyl, propyl, or a polyether group [-O(R⁶-O)ₓ-].

28. A surfactant composition according to any one of claims 1 to 17, **characterized in that** the gemini surfactant has the general formula (D.I) 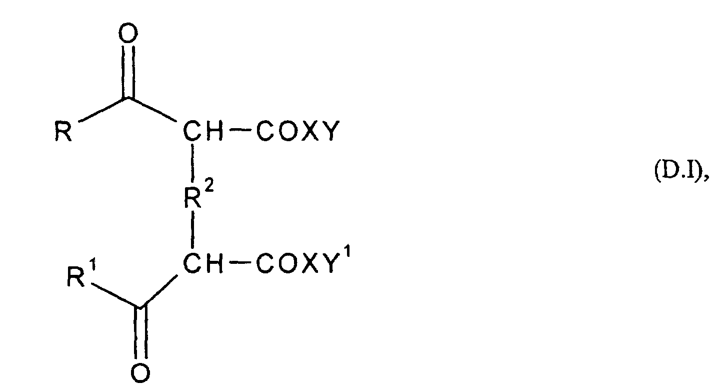 wherein the substituents have the following meanings:
**R, R**¹ C₅- to C₃₀-alkyl, branched or unbranched, saturated, optionally as far as non-adjacently diunsaturated, hydroxy-substituted or perfluorinated;
**R**² C₁- to C₁₀-alkylene, arylene, or the hydroxy-substituted derivatives thereof, a polyether [-O(R⁴O)ₓ-], -S-, -SO -O-, -S-S-, -O-R⁵-O-, or -S-R⁵-S-; variable for a direct bond between the two α-carbons;
**R**⁴ C₂- to C₄-alkylene;
**R**⁵ C₁- to C₁₀-alkylene, arylene, or alkylarylene, -N(R⁶)-, or -(NR⁶)R⁷-(NR⁶)-;
**R**⁶ C₁- to C₆-alkyl;
**R**⁷ C₁- to C₆-alkyl, wherein R⁷ and R⁶ can also be part of a heterocyclic ring;
**X** polyether [-O(R⁴O)ₓ-], wherein x is a number from 1 to 30, -O-, NZ;
**Z** C₁- to C₁₀-alkyl, aryl, alkylaryl, or H, and
**Y, Y**¹ are independently H, -CH₂-COOH and salts, a hydrocarbon moiety having at least two hydroxyl groups, such as erythrose, threose, ribose, arabinose, xylose, fructose, lyxose, allose, altrose, glucose, mannose, galactose, and mixtures thereof.

29. A surfactant composition according to any one of claims 1 to 17, **characterized in that** the gemini surfactant has the general formula (D.II) 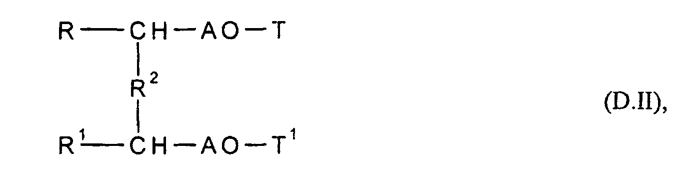 wherein the substituents have the meanings as defined by the general formula (D.I) of claim 28 and
**AO** is -C(O)-, -C(O)- [-O(R⁴O)ₓ-], -CH₂-[-O(R⁴O)ₓ-], -CH₂-O-;
**T, T**¹ are independently -OM, -H, -CH₃, -C₂H₅, -SO₃M, -CH₂COOM, -C₂H₄COOM, -C₃H₆-SO₃M, -O-P(O)(OM)₂ and
**M** is alkali, ½ alkaline earth, ammonium, mono-, di-, trialkanolammonium, or H.

30. A surfactant composition according to any one of claims 1 to 17, **characterized in that** the gemini surfactant has the general formula (D.III) 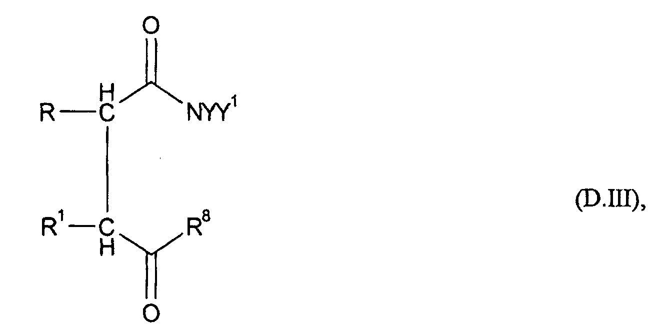 wherein the substituents have the meanings as defined by the general formulas (D.I) and (D.II) according to claims 28 and 29 and
**R**⁸ represents NYY¹, -O(R⁴O)ₓH or -O(R⁴O)ₓ-C(O)-CHR-CHR¹-C(O)NYY¹.

31. A surfactant composition according to any one of claims 1 to 17, **characterized in that** the gemini surfactant has the general formula (D.IV) 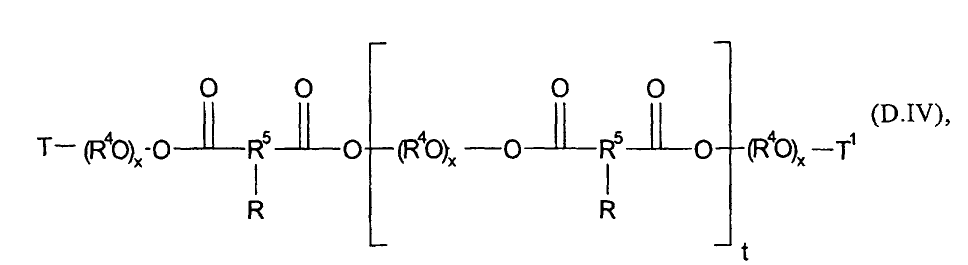 wherein the substituents have the meanings as defined by the general formulas (D.I), (D.II), and (D.III) according to claims 28 to 30 and
**t** is an integer from 1 to 100, preferably 1 to 20, most preferably 1 to 4.

32. The use of the surfactant composition as claimed in any one of the preceding claims as an emulsifier or dispersant, an additive for conventional anionic surfactants, or an additive for skin and hair cleaning preparations.

## Revendications

1. Composition tensioactive contenant
(A) à raison de 1 à 70 % en poids, de préférence de 10 à 60 % en poids par rapport aux composants (A) et (B), un ou plusieurs tensioactifs géminés et
(B) pour le reste, rapportés à la somme des composants (A) et (B), plusieurs co-amphiphiles, les co-amphiphiles présentant une valeur HLB inférieure ou égale à 6 et étant choisis parmi au moins 2 des composants amphiphiles sous-mentionnés (a) à (d) :
(a) un ou plusieurs alcools à chaîne plus longue :
- un alcool en C₆ à C₄₀,
(b) un ou plusieurs acides à chaîne plus longue :
- un acide carboxylique en C₆ à C₂₄,
(c) un ou plusieurs esters/esters partiels d'un polyol avec un ou plusieurs acides mono- ou polycarboxyliques :
- un ester en C₆ à C₂₂ de sorbitane,
- un ester en C₆ à C₂₂ de méthylglucoside,
- un ester en C₆ à C₂₂ de sucre,
- un mono-, di- et triglyceride d'un acide carboxylique en C₆ à C₂₂,
- un dérivé du mono- et diglyceride d'un acide carboxylique en C₆ à C₂₂, estérifié avec l'acide lactique ou l'acide citrique,
- un ester en C₆ à C₂₂ de polyglycérol,
- un ester en C₆ à C₂₂ de polypropylèneglycol et/ou
- un ester de vitamine,
(d) ainsi que les autres composants co-amphiphiles suivants :
- l'acide salicylique,
- l'acide benzoïque et/ou
- la lécithine,
au moins un co-amphiphile étant
- un alcool en C₆ à C₄₀ et/ou
- un mono-, di- et triglyceride d'un acide carboxylique en C₆ à C₂₂.

2. Composition tensioactive selon la revendication 1, **caractérisée en ce que** la composition tensioactive contient
(A) à raison de 5 à 60 % en poids, de préférence de 10 à 50 % en poids par rapport aux composants (A) et (B), un ou plusieurs tensioactifs et
(B) pour le reste, rapportés à la somme des composants (A) et (B), un ou plusieurs co-amphiphiles.

3. Composition tensioactive selon l'une quelconque des revendications précédentes, contenant en outre
(C) à raison d'au moins 0,1 % en poids, par rapport à la composition totale, de l'eau.

4. Composition tensioactive selon l'une quelconque des revendications précédentes, contenant en outre
(D) à raison d'au moins 0,1 % en poids, par rapport à la composition totale, un ou plusieurs composants d'huile.

5. Composition tensioactive selon l'une quelconque des revendications précédentes, dans laquelle au moins un co-amphiphile est
- un acide carboxylique en C₆ à C₂₄, de préférence en C₈ à C₂₂ et/ou
- un dérivé du mono- et diglyceride d'un acide carboxylique en C₆ à C₂₂, estérifié avec l'acide lactique ou l'acide citrique.

6. Composition tensioactive selon l'une quelconque des revendications précédentes sous forme d'une émulsion, **caractérisée en ce que** le co-amphiphile se présente à 25 °C à l'état solide.

7. Composition tensioactive sous forme d'une dispersion selon l'une quelconque des revendications 1 à 5, **caractérisée en ce que** le co-amphiphile se présente à 25 °C à l'état liquide.

8. Composition tensioactive selon l'une quelconque des revendications précédentes, **caractérisée en ce que** 3 à 5 types différents de co-amphiphiles sont utilisés.

9. Composition tensioactive
selon l'une quelconque des revendications précédentes, dans laquelle au moins un co-amphiphile est
- un alcool en C₆ à C₄₀, de préférence un alcool en C₈ à C₂₄.

10. Composition tensioactive selon l'une quelconque des revendications précédentes, **caractérisée en ce que** la composition tensioactive contient
- 30 à 50 % en poids d'un alcool en C₆ à C₄₀, de préférence en C₈ à C₂₄ et/ou
- 30 à 50 % en poids d'un mono-, di-, et triglyceride d'un acide carboxylique en C6 à C22,
à chaque fois. par rapport à la composition tensioactif géminé/co-amphiphile(s).

11. Composition tensioactive selon l'une quelconque des revendications précédentes sous forme d'une émulsion, **caractérisée en ce que** la composition tensioactive peut être préparée par un procédé (méthode de la température de changement de phase (PTT)), lequel présente les étapes suivantes :
réunion
(a) d'une composition (a) contenant le ou constituée du tensioactif géminé (A) ainsi qu'éventuellement d'eau, la composition présentant une température X, avec
(b) une composition (b) contenant le ou constituée du co-amphiphile (B) ainsi le cas échéant d'un composant d'huile, la composition présentant une température Y,
la température Y étant supérieure à la température X.

12. Composition tensioactive selon la revendication 11, **caractérisée en ce que** la température Y est supérieure d'au maximum 15°C à la température critique de changement de phase du tensioactif dans la composition (b).

13. Compositions tensioactives selon l'une quelconque des revendications 11 ou 12, **caractérisées en ce que** les températures X et Y diffèrent d'au moins 3°C, de préférence d'au moins 5°C.

14. Composition tensioactive selon l'une quelconque des revendications précédentes, **caractérisée en ce que** le tensioactif géminé présente à l'endroit de liaison entre espaceur, groupement hydrophile et groupement hydrophobe des atomes d'azote.

15. Composition tensioactive selon l'une quelconque des revendications précédentes, **caractérisé en ce que** le tensioactif géminé présente un espaceur présentant 1 à 12 atomes de carbone et des groupements amine ou amide.

16. Composition tensioactive selon l'une quelconque des revendications précédentes, **caractérisée en ce que** les deux groupements hydrophobes présentent chacun un radical hydrocarboné en C₆ à C₂₄ et/ou le groupement (de tête) double hydrophile présente un radical alcoxylé au moins une fois qui porte un groupement acide sulfonique, acide carboxylique, acide phosphonique, polyalcool ou poly(oxyde d'alkylène) lequel peut se présenter sous une forme saline.

17. Composition tensioactive selon l'une quelconque des revendications précédentes, **caractérisée en ce que** la composition tensioactive contient 0,01 à 30 % en poids, de préférence 0,1 à 6 % en poids des composants (A) et (B) par rapport à la composition totale.

18. Composition tensioactive selon l'une quelconque des revendications 1 à 17, **caractérisée en ce que** le tensioactif géminé présente la formule générale (A.I) 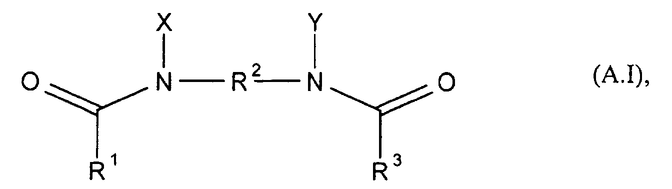 dans laquelle les substituants ont la signification suivante :
R¹,R³ est un alkyle en C₅ à C₂₅, ramifié ou non ramifié, saturé, le cas échéant jusqu'à deux fois insaturé non successivement ;
R² un alkylène en C₁ à C₁₂
X,Y (C₂H₄O-)ₓ(C₃H₆O-)_{y}-FR ; x+y ≥ 1, x :0-15, y :0-10 et
FR -SO₃M, -CH₂-CO₂M, -P(O) (OM)₂, H, -C₃H₆SO₃M, -CH₂(CHOH)₄CH₂OH pour autant que x+y = 0, M = métal alcalin, (alkyl)ammonium, alcanolammonium, H ou ½ métal alcalinoterreux

19. Composition tensioactive selon l'une quelconque des revendications 1 à 17, **caractérisée en ce que** le tensioactif géminé présente la formule générale (A.II) 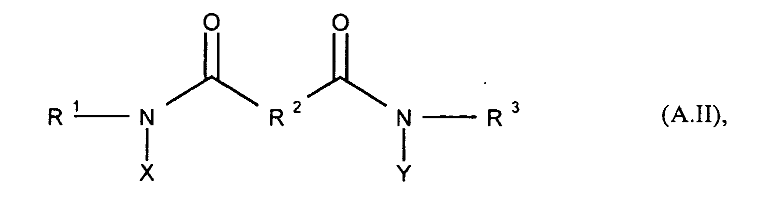 dans laquelle les substituants ont la signification indiquée pour la formule générale (A.I) à la revendication 18.

20. Composition tensioactive selon l'une quelconque des revendications 1 à 17, **caractérisée en ce que** le tensioactif géminé présente la formule générale (A.III) 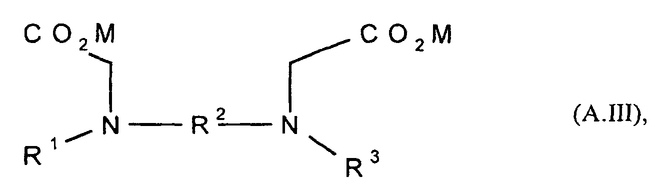 dans laquelle les substituants ont la signification indiquée pour la formule générale (A.I) à la revendication 18.

21. Composition tensioactive selon l'une quelconque des revendications 1 à 17, **caractérisée en ce que** le tensioactif géminé présente la formule générale (B.I) 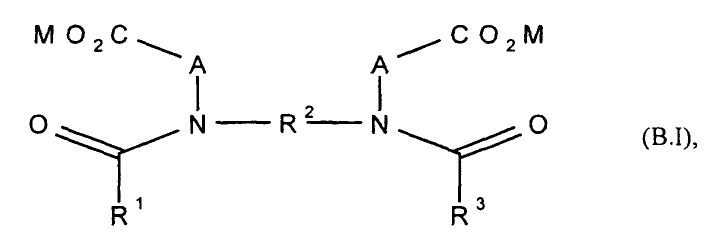 dans laquelle les substituants ont la signification suivante :
R¹, R³ est un alkyle en C₅ à C₂₅, ramifié ou non ramifié, saturé, le cas échéant jusqu'à deux fois insaturé non successivement ;
R² un alkylène en C₁ à C₁₂ ;
A CHR⁴, CH₂, C₂H₄, C₃H₆, C₄H₈ ;
R⁴ un radical d'un acide aminé et
M un métal alcalin, (alkyl)ammonium, alcanolammonium, H ou ½ métal alcalinoterreux

22. Composition tensioactive selon l'une quelconque des revendications 1 à 17, **caractérisée en ce que** le tensioactif géminé présente la formule générale (B.II) 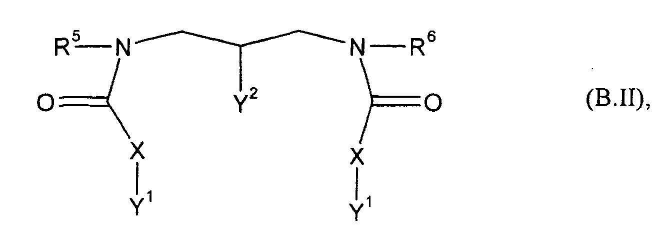 dans la quelle les substituants ont la signification indiquée à la revendication 21 pour la formule générale (B.I) et
R⁵, R⁶ signifie un alkyle en C₆ à C₃₆, ramifié ou non ramifié, saturé, le cas échéant jusqu'à deux fois insaturé non successivement ;
X signifie un groupement alkylène ou alcénylène présentant 1 à 6 atomes de carbone et éventuellement substitué par un groupement hydroxyle ou un groupement acide sulfonique ou un groupement carboxy ;
Y¹ signifie un groupement sulfonate ou sulfate ou un groupement carboxyle et
Y² signifie un groupement hydroxyle, un radical acide sulfurique ou -O-C(O)X-COOH

23. Composition tensioactive selon l'une quelconque des revendications 1 à 17, **caractérisée en ce que** le tensioactif géminé présente la formule générale (B.III) 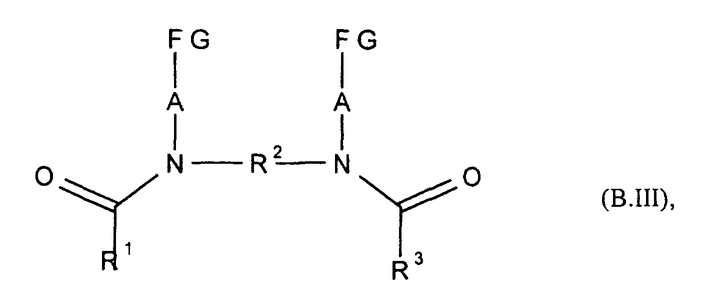 dans laquelle les substituants ont la signification indiquée pour la formule générale (B.I) à la revendication 21 et
FG signifie -COOM ou-SO₃M

24. Composition tensioactive selon l'une quelconque des revendications 1 à 17, **caractérisée en ce que** le tensioactif géminé présente la formule générale (B.IV) 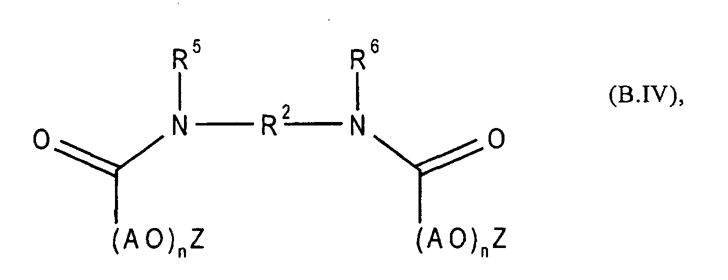 dans laquelle les substituants ont la signification indiquée aux formules générales (B.I) et (B.II) et
AO signifie des unités oxyde d'alkylène, c'est-à-dire des unités éthylèneglycol, propylèneglycol, éther butylèneglycolique, seules ou réparties statistiquement ou en blocs, avec n = 1 à 20 et
Z signifie -SO₃M, -C₂H₄SO₃M, -C₃H₆SO₃M, -P(O)(OM)₂, -CH₂-COOM ou -C₂H₄-COOM.

25. Composition tensioactive selon l'une quelconque des revendications 1 à 17, **caractérisée en ce que** le tensioactif géminé présente la formule générale (C.I) 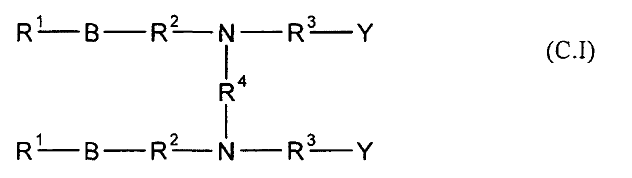 dans laquelle les substituants ont la signification suivante :
R¹ est un alkyle en C₅ à C₂₅, ramifié ou non ramifié, saturé, le cas échéant jusqu'à deux fois insaturé non successivement, substitué par hydroxy ou perfluoré ;
R² un alkylène en C₁ à C₁₂ ou ses dérivés substitués par hydroxy ;
B un groupement amide [-C(O)N(R²)- ou -N(R⁵)C(O)-], un groupement carboxylique [-C(O)O- ou -OC(O)-], un groupement polyéther [-(O(R⁶-O)ₓ-] ;
R⁵ un alkyle en C₁ à C₄ ou un alkyle substitué par hydroxy, ou un H ;
R⁶ un alkylène en C₂ à C₄ ;
x un nombre de 1 à 20 ;
R³ un alkyle en C₁ à C₁₂ ou ses dérivés substitués par hydroxy, R⁷-D-R⁷ ou un groupement polyéther [-(O(R⁶-O)ₓ-] ;
R⁷ un alkylène en C₁ à C₆ ou ses dérivés substitués par hydroxy ;
D -O-, -S-, -N(R⁸)- ;
R⁴ un alkylène ou un alkylaryle avec 1 à 12 atomes de C ou les dérivés substitués par hydroxy ou R⁹-D¹-R⁹ ;
R⁸ un alkyle en C₁ à C₁₂ ou un alkyle substitué par hydroxy, ou un H ou un R⁹-D¹-R⁹ ;
R⁹ un alkylène en C₁ à C₆ ou ses dérivés substitués par hydroxy ou un aryle ;
D¹ -O-, -S-, -SO₂-, -C(O)-, [-O(R⁷-O)ₓ-], (R¹⁰)ₜ[N(R¹⁰)]_{z} ou aryle
R¹⁰ un alkyle en C₁ à C₁₂ ou un alkyle substitué par hydroxy, ou un H ou un aryle;
t,z signifient indépendamment l'un de l'autre un nombre de 1 à 4 et
Y sont indépendamment l'un de l'autre -SO₃H , -O-SO₃H, -OP(O)(OH)₂, -COOH, -CO₂-C₆H₄-SO₃H ou leurs sels

26. Composition tensioactive selon l'une quelconque des revendications 1 à 17, **caractérisée en ce que** le tensioactif géminé présente la formule générale (C.II) 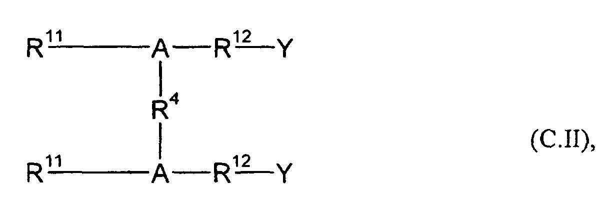 dans laquelle les substituants ont la signification indiquée pour la formule générale (C.I) à la revendication 25 et
R¹¹ signifie un alkyle en C₅ à C₂₃, ramifié ou non ramifié, saturé, le cas échéant jusqu'à deux fois insaturé non successivement, substitué par hydroxy ou perfluoré ou R¹⁴-B-R² ;
R¹⁴ un alkyle en C₁ à C₁₂, ramifié ou non ramifié, saturé, le cas échéant jusqu'à deux fois insaturé non successivement, ou les dérivés substitués par hydroxy ;
R¹² un alkylène en C₁ à C₁₂, ramifié ou non ramifié, saturé, le cas échéant jusqu'à deux fois insaturé non successivement, ou les dérivés substitués par hydroxy ou une groupement amide [-C(O)N(R²)- ou -N(R⁵)C(O)-], un groupement carboxyle [-C(O)O ou -OC(O)-]; un groupement polyéther [-O(R⁶-O)ₓ-] ou R⁹-D¹-R⁹ et
A -CR⁶= ou -N= à condition que lorsque A est identique à -N=, R¹¹ est identique à R¹⁴-B-R²

27. Composition tensioactive selon l'une quelconque des revendications 1 à 17, **caractérisée en ce que** le tensioactif géminé présente la formule générale (C.III) 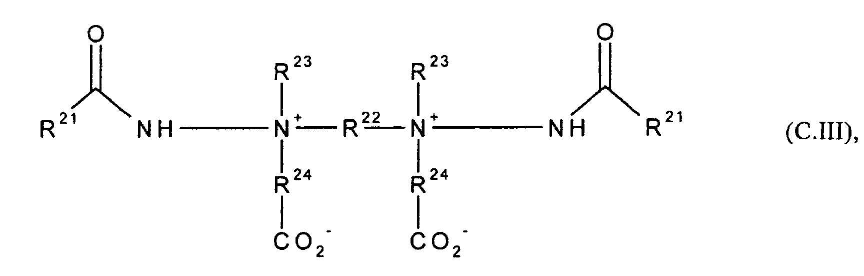 dans laquelle les substituants ont la signification indiquée pour les formules générales (C.I) et (C.II) et
R²¹ signifie un alkyle en C₅ à C₂₃, ramifié ou non ramifié, saturé, le cas échéant jusqu'à deux fois insaturé non successivement ;
R²², R²⁴ un alkylène en C₁ à C₆ et
R²³ un méthyle, éthyle, propyle ou un groupement polyéther [-(O(R⁶-O)ₓ-]

28. Composition tensioactive selon l'une quelconque des revendications 1 à 13, 16 ou 17 **caractérisée en ce que** le tensioactif géminé présente la formule générale (D.I) 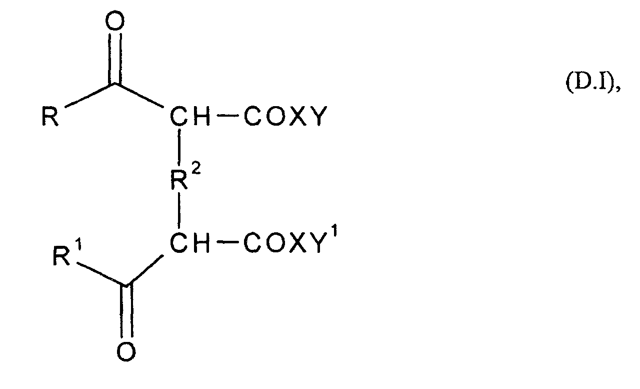 dans laquelle les substituants ont la signification suivante :
R,R¹ signifie un alkyle en C₅ à C₃₀, ramifié ou non ramifié, saturé, le cas échéant jusqu'à deux fois insaturé non successivement, substitué par hydroxy ou perfluoré ;
R² un alkylène en C₁ à C₁₀, un arylène ou leurs dérivés substitués par hydroxy, un polyéther [-O(R⁴O)ₓ-], -S-, -SO₂-, -O-, -S-S-, -O-R⁵-O- ou -S-R⁵-O- ou-S-R⁵-S- ; variable pour une liaison directe entre les deux carbones en α ;
R⁴ un alkylène en C₂ à C₄ ;
R⁵ un alkylène en C₁ à C₁₀, un arylène ou alkylarylène, -N(R⁶)- ou -(NR⁶)-R⁷-(NR⁶) ;
R⁶ un alkyle en C₁ à C₆ ;
R⁷ un alkyle en C₁ à C₆, R⁷ et R⁶ pouvant également faire partie d'un noyau hétérocyclique ;
X un polyéther [-O(R⁴O)x-] où x est un nombre de 1 à 30, -O-, NZ ;
Z un alkyle en C₁ à C₁₀, un aryle, un alkylaryle ou H et
Y,Y¹ indépendamment l'un de l'autre H, -CH₂-COOH et sels, un radical hydrocarboné présentant au moins deux groupements hydroxyle tel que erythrose, thréose, ribose, arabinose, fructose, xylose, lyxose, allose, altrose, glucose, mannose, galactose et leurs mélanges.

29. Composition tensioactive selon l'une quelconque des revendications 1 à 13, 16 ou 17 **caractérisée en ce que** le tensioactif géminé présente la formule générale (D.II) 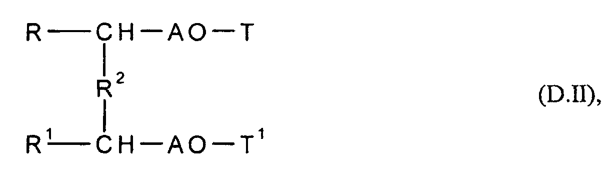 dans laquelle les substituants ont la signification indiquée à la revendication 28 pour la formule générale (D.I) et
AO signifie -C(O)-, -C(O)-[-O(R⁴O)ₓ-], -CH2-[-O(R⁴O)ₓ-], -CH₂-O-;
T,T¹ indépendamment l'un de l'autre -OM, -H, -CH₃, -C₂H₅, -SO₃M, -CH₂COOM, -C₂H₄-COOM, -C₃H₆-SO₃M, -O-P(O) (OM)₂ et
M un métal alcalin, ½ métal alcalinoterreux, ammonium, mono-, di-, trialcanolammonium ou H

30. Composition tensioactive selon l'une quelconque des revendications 1 à 17, **caractérisée en ce que** le tensioactif géminé présente la formule générale (D.III) 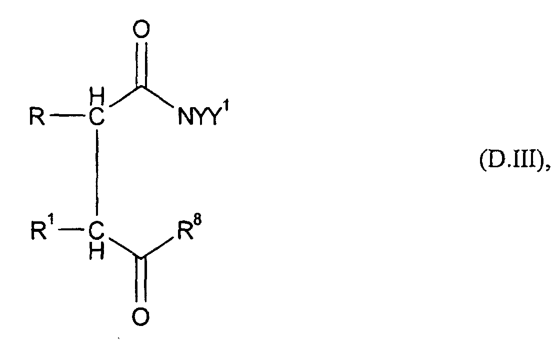 dans laquelle les substituants ont la signification indiquée pour les formules générales (D.I) et (D.II) et
R8 signifie NYY', -O(R⁴O)ₓH ou -O(R⁴O)ₓ-C(O)-CHR-CHR¹-C(O)NYY¹.

31. Composition tensioactive selon l'une quelconque des revendications 1 à 13, 16 ou 17 **caractérisée en ce que** le tensioactif géminé présente la formule générale (D.IV) 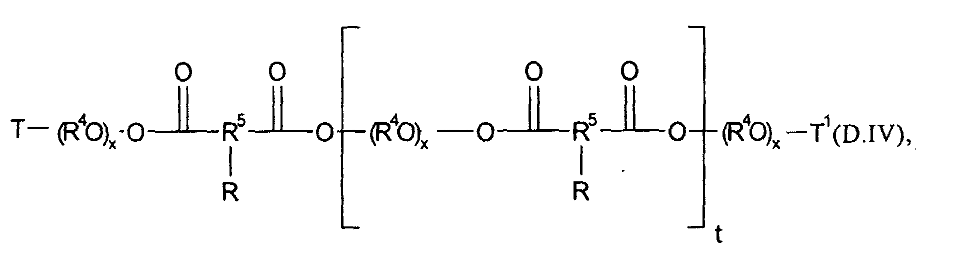 dans laquelle les substituants ont la signification indiquée pour les formules générales (D.I), (D.II) et (D.III) et
t signifie un nombre entier de 1 à 100, de préférence de 1 à 20, de manière particulièrement préférée de 1 à 4.

32. Utilisation de la composition tensioactive selon l'une quelconque des revendications précédentes comme émulsionnant ou dispersant, comme additif à des tensioactifs anioniques conventionnels ou comme additif à des formulations pour le lavage des cheveux et de la peau.
